# EUROPEAN PATENT APPLICATION

(11) **EP 4 227 409 A1**
(43) Date of publication of application: **16.08.2023**
(21) Application number: 21877718.3
(22) Date of filing: 07.10.2021
(51) Int. Cl.: C12N 15/09, C12N 1/15, C12N 1/19, C12N 1/21, C12N 5/10, C12N 9/22, C12N 15/55, C12N 15/62, C12N 15/63

(54) **TECHNIQUE FOR MODIFYING TARGET NUCLEOTIDE SEQUENCE USING CRISPR-TYPE I-D SYSTEM**

(30) Priority: 08.10.2020 JP 2020170714
(71) Applicant: Tokushima University, Tokushima-shi, Tokushima 770-8501 (JP)
(72) Inventor: OSAKABE, Keishi, Tokushima-shi, Tokushima 770-8501 (JP); OSAKABE, Yuriko, Tokushima-shi, Tokushima 770-8501 (JP); WADA, Naoki, Tokushima-shi, Tokushima 770-8501 (JP)
(74) Representative: dompatent von Kreisler Selting Werner - Partnerschaft von Patent- und Rechtsanwälten mbB
(86) International application number: PCT/JP2021/037194
(87) International publication number: WO 2022/075419

(57) **Abstract**

Provided are a method for targeting a target nucleotide sequence, etc. said method comprising introducing into a cell: (i) CRISPR type I-D Cas proteins Cas5d, Cas6d and Cas7d and a polypeptide containing the N-terminal HD domain of Cas10d, or nucleic acids encoding the proteins and the polypeptide; (ii) a polypeptide that contains not the N-terminal HD domain of Cas10d but the C-terminal partial sequence of Cas10d, or a nucleic acid encoding the polypeptide; and (iii) a crRNA that contains a sequence capable of forming a base pair with the target nucleotide sequence, or a DNA encoding the crRNA.

## Description

### Technical Field

The present invention relates to a method for targeting a target nucleotide sequence, a method for specifically altering a target nucleotide sequence, and a method for suppressing the expression of a target gene, wherein these methods utilize CRISPR (Clustered Regularly Interspaced Short Palindromic Repeats) type I-D system, and a complex and a kit comprising Cas (CRISPR-associated) proteins and a crRNA (CRISPR RNA) used for the methods, etc.

### Background Art

CRISPR-Cas systems are adaptive immune systems found in bacteria and archaea, which defend bacteria and archaea from viruses, plasmids and other foreign genetic elements. CRISPR-Cas systems are classified into two classes consisting of six different types (I-VI) and at least 34 subtypes based on different Cas proteins constituting the systems and different molecular mechanisms for the systems.

In mechanisms for type I and II systems, complexes of a crRNA and Cas effector proteins recognize short (typically 3 to 5 nucleotides in length) sequence elements which are called PAMs (protospacer adjacent motifs). After recognition of PAMs, the type I and II crRNA-Cas effector protein complexes locally disrupt base pairs in target DNAs to form R-loop structures, and then the crRNA guide elements form base pairs with the complementary target strands to replace the non-target DNA strands. Binding and unwinding of the target double stranded DNA by the crRNA-Cas complex are required for DNA cleavage and DNA degradation by type-specific Cas effector nucleases such as Cas3, Cas9 and Cas12 nucleases.

For CRISPR type I, there are various subtypes. Class 1 systems include target recognition modules such as Cas5, Cas6, Cas7, and Cas8, termed Cascade (CRISPR-associated-complex for antiviral defense), and a DNA cleavage module such as Cas3 (see Non-patent Literature 1 and Non-patent Literature 2). For genome editing techniques, Cass 1 CRISPR systems have been less common than Class 2 CRISPR systems. However, it has been suggested that Cass 1 CRISPR systems may have some advantages as compared with Cas9 and Cpf1 (see Non-patent Literature 1, and Patent Literature 1). For example, Cass 1 CRISPR systems have various mutation profiles including long-range genome deletion and long gRNA sequences. It has been previously reported that a Class 1 type I-E system induces base deletion of 2-300 b to 100 kb mainly 5' upstream of PAM sequences (see Non-patent Literature 3).

The Class 1 CRISPR type I-E system as previously studied is composed of six Cas proteins (Cas3e, Cas5e, Cas6e, Cas7e, Cas8e, and Cas11e), and a crRNA for targeting. Cas8e and Cas11e are called a large subunit and a small subunit, respectively, and are believed to function as support proteins for stably maintaining the binding between the Cas protein complex and the target DNA (see Non-patent Literature 1) .

On the other hand, the present inventors previously identified a CRISPR-Cas genomic locus encoding a Class 1 type I subtype system, named CRISPR type I-D (hereinafter, referred to as "TiD"), and then found that the system can do genome editing by using five Cas proteins of Cas3d, Cas5d, Cas6d, Cas7d and Cas10d and a crRNA for targeting (see Patent Literature 1 and Patent Literature 2). In the TiD locus, a gene corresponding to Cas11e, which is a small subunit for the CRISPR type I-E system, was not found.

In recent years, McBride et al. analyzed the TiD locus in *Synechocystis* sp. PCC6803, and found that there are a ribosome binding site (RBS) and a translation start codon within the Cas10d locus and two proteins of Cas10d and Cas11d are translated from the Cas10d locus using a common stop codon (see Non-patent Literature 4). However, the function of Cas11d for target sequence-alteration techniques has never been found.

### Citation List

### Patent Literature

Patent Literature 1: WO2019/039417
Patent Literature 2: WO2020/184723

### Non-patent Literature

Non-patent Literature 1: Makarova K.S., et al., Nat. Rev. Microbiol., 13, 722-736 (2015)
Non-patent Literature 2: Makarova, K.S., et al., Cell, 168, 946-946 (2017)
Non-patent Literature 3: Dolan, A.E. eta l., Mol Cell 74, 936-950 (2019)
Non-patent Literature 4: McBride, T.M. et al., bioRxiv, doi: https://doi.org/10.1101/2020. 04. 18. 045682 (2020)

### Summary of Invention

### Technical Problems

An objective of the present invention is to improve the targeting efficiency and alteration efficiency of a target sequence by TiD.

### Solution to Problems

As a result of intensive study, the present inventors surprisingly have found that a target nucleotide sequence can be efficiently altered by expressing a polypeptide comprising a partial amino acid sequence containing a C-terminal region of Cas10d, in addition to the five Cas proteins constituting the TiD system which were previously reported. Thus the present invention was completed.

That is, the present invention provides:
[1] A method for targeting a target nucleotide sequence, the method comprising introducing into a cell:
   (i) CRISPR type I-D Cas proteins Cas5d, Cas6d and Cas7d, and a polypeptide containing the N-terminal HD domain of Cas10d, or nucleic acids encoding the proteins and the polypeptide,
   (ii) a polypeptide that does not contain the N-terminal HD domain of Cas10d and contains a C-terminal partial sequence of Cas10d, or a nucleic acid encoding the polypeptide, and
   (iii) a crRNA containing a sequence capable of forming a base pair with the target nucleotide sequence, or a DNA encoding the crRNA;
[2] A method for altering a target nucleotide sequence, the method comprising introducing into a cell:
   (i) CRISPR type I-D Cas proteins Cas3d, Cas5d, Cas6d and Cas7d, and a polypeptide containing the N-terminal HD domain of Cas10d, or nucleic acids encoding the proteins and the polypeptide,
   (ii) a polypeptide that does not contain the N-terminal HD domain of Cas10d and contains a C-terminal partial sequence of Cas10d, or a nucleic acid encoding the polypeptide, and
   (iii) a crRNA containing a sequence capable of forming a base pair with the target nucleotide sequence, or a DNA encoding the crRNA;
[3] The method according to [1] or [2], which is for regulating the transcription of a target gene, and wherein the target nucleotide sequence is at least a partial sequence of the target gene;
[4] The method according to any one of [1] to [3], wherein the C-terminal partial sequence of Cas10d is a sequence consisting of 100 to 400 amino acids;
[5] The method according to any one of [1] to [4], wherein the cell is a eukaryotic cell;
[6] The method according to any one of [2] to [5], wherein the alteration is nucleotide deletion, insertion or substitution;
[7] A complex comprising:
   (i) CRISPR type I-D Cas proteins Cas5d, Cas6d and Cas7d, and a polypeptide containing the N-terminal HD domain of Cas10d,
   (ii) a polypeptide that does not contain the N-terminal HD domain of Cas10d and contains a C-terminal partial sequence of Cas10d, and
   (iii) a crRNA containing a sequence capable of forming a base pair with the target nucleotide sequence;
[8] The complex according to [7], further comprising Cas3d;
[9] The complex according to [7] or [8], wherein the C-terminal partial sequence of Cas10d is a sequence consisting of 100 to 400 amino acids;
[10] A vector containing:
   (i) nucleic acids encoding CRISPR type I-D Cas proteins Cas5d, Cas6d and Cas7d and a polypeptide containing the N-terminal HD domain of Cas10d,
   (ii) a nucleic acid encoding a polypeptide that does not contain the N-terminal HD domain of Cas10d and contains a C-terminal partial sequence of Cas10d, and
   (iii) a crRNA containing a sequence capable of forming a base pair with the target nucleotide sequence, or a DNA encoding the crRNA;
[11] The vector according to [10], further containing a nucleic acid encoding Cas3d;
[12] The expression vector according to [10] or [11], wherein the nucleic acids of (i) to (iii), or the nucleic acids of (i) to (iii) and the nucleic acid encoding Cas3d are contained in a single vector or two or more vectors;
[13] The vector according to [11] or [12], wherein the C-terminal partial sequence of Cas10d is a sequence consisting of 100 to 400 amino acids;
[14] A DNA molecule encoding the complex according to any one of [7] to [9];
[15] A kit for targeting a target nucleotide sequence comprising:
   (i) CRISPR type I-D Cas proteins Cas5d, Cas6d and Cas7d, and a polypeptide containing the N-terminal HD domain of Cas10d, or nucleic acids encoding the proteins and the polypeptide,
   (ii) a polypeptide that does not contain the N-terminal HD domain of Cas10d and contains a C-terminal partial sequence of Cas10d, or a nucleic acid encoding the polypeptide, and
   (iii) a crRNA containing a sequence capable of forming a base pair with the target nucleotide sequence, or a DNA encoding the crRNA;
[16] A kit for altering a target nucleotide sequence comprising:
   (i) CRISPR type I-D Cas proteins Cas3d, Cas5d, Cas6d and Cas7d, and a polypeptide containing the N-terminal HD domain of Cas10d, or nucleic acids encoding the proteins and the polypeptide,
   (ii) a polypeptide that does not contain the N-terminal HD domain of Cas10d and contains a C-terminal partial sequence of Cas10d, or a nucleic acid encoding the polypeptide, and
   (iii) a crRNA containing a sequence capable of forming a base pair with the target nucleotide sequence, or a DNA encoding the crRNA;
[17] The kit according to [15] or [16], wherein the C-terminal partial sequence of Cas10d is a sequence consisting of 100 to 400 amino acids;
[18] A method for improving targeting efficiency in targeting a target nucleotide sequence using a CRISPR type I-D system, the method comprising using a polypeptide that does not contain the N-terminal HD domain of Cas10d and contains a C-terminal partial sequence of Cas10d, or a nucleic acid encoding the polypeptide;
[19] A method for improving alternation efficiency in altering a target nucleotide sequence using a CRISPR type I-D system, the method comprising using a polypeptide that does not contain the N-terminal HD domain of Cas10d and contains a C-terminal partial sequence of Cas10d, or a nucleic acid encoding the polypeptide;
[20] A composition for improving targeting efficiency in targeting a target nucleotide sequence using a CRISPR type I-D system, comprising a polypeptide that does not contain the N-terminal HD domain of Cas10d and contains a C-terminal partial sequence of Cas10d, or a nucleic acid encoding the polypeptide;
[21] A composition for improving alteration efficiency in altering a target nucleotide sequence using a CRISPR type I-D system, comprising a polypeptide that does not contain the N-terminal HD domain of Cas10d and contains a C-terminal partial sequence of Cas10d, or a nucleic acid encoding the polypeptide;
[22] A method for producing a cell comprising an altered target nucleotide sequence, the method comprising introducing into a cell:
   (i) CRISPR type I-D Cas proteins Cas3d, Cas5d, Cas6d and Cas7d, and a polypeptide containing the N-terminal HD domain of Cas10d, or nucleic acids encoding the proteins and the polypeptide,
   (ii) a polypeptide that does not contain the N-terminal HD domain of Cas10d and contains a C-terminal partial sequence of Cas10d, or a nucleic acid encoding the polypeptide, and
   (iii) a crRNA containing a sequence capable of forming a base pair with the target nucleotide sequence, or a DNA encoding the crRNA;
[23] A method for producing a plant comprising an altered target nucleotide sequence, the method comprising producing a plant cell comprising the altered target nucleotide sequence by the method according to [22];
[24] A method for producing a non-human animal comprising an altered target nucleotide sequence, the method comprising producing a non-human animal cell comprising the altered target nucleotide sequence by the method according to [22];
[25] A method for targeting a target nucleotide sequence, the method comprising bringing:
   (i) CRISPR type I-D Cas proteins Cas5d, Cas6d and Cas7d, and a polypeptide containing the N-terminal HD domain of Cas10d,
   (ii) a polypeptide that does not contain the N-terminal HD domain of Cas10d and contains a C-terminal partial sequence of Cas10d, and
   (iii) a crRNA containing a sequence capable of forming a base pair with the target nucleotide sequence, or a DNA encoding the crRNA,
   into contact with an isolated nucleic acid comprising the target nucleotide sequence; and
[26] A method for altering a target nucleotide sequence, the method comprising bringing:
   (i) CRISPR type I-D Cas proteins Cas3d, Cas5d, Cas6d and Cas7d, and a polypeptide containing the N-terminal HD domain of Cas10d,
   (ii) a polypeptide that does not contain the N-terminal HD domain of Cas10d and contains a C-terminal partial sequence of Cas10d, and
   (iii) a crRNA containing a sequence capable of forming a base pair with the target nucleotide sequence, or a DNA encoding the crRNA,
   into contact with an isolated nucleic acid comprising the target nucleotide sequence.

### Effects of the Invention

According to the present invention, site-specific mutations can be efficiently induced in cells, preferably animal and plant cells, by using a TiD system comprising a TiD crRNA engineered to target a specific DNA. Surprisingly, according to the present invention, the efficiency of targeting and altering a target sequence by the TiD system can be increased several times by expressing a C-terminal partial sequence of Cas10d (hereinafter also referred to as "Cas10d C-ter") in addition to expressing TiD system Cas effector proteins Cas3d, Cas5d, Cas6d, Cas7d and Cas10d. Further, the technique of the present invention induces longer-range deletion as a mutation near the target sequence.

### Brief Description of Drawings

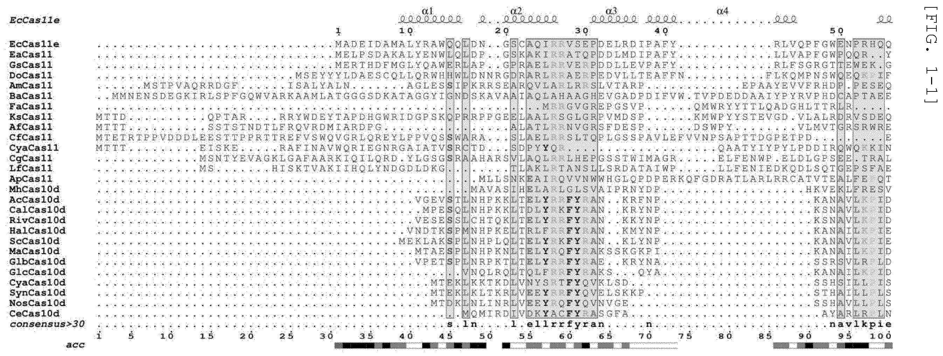

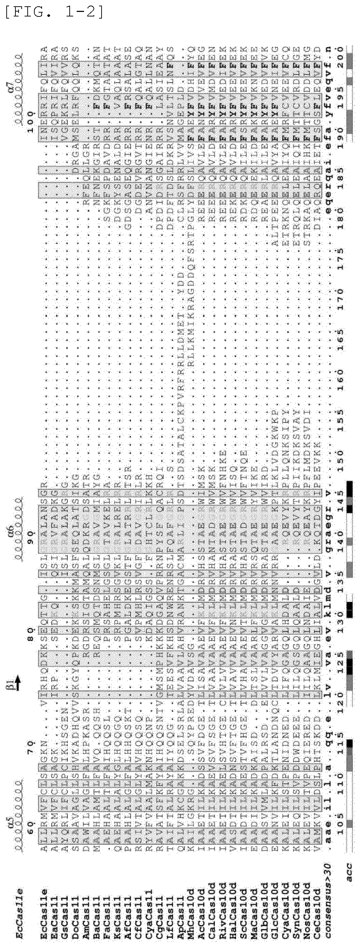

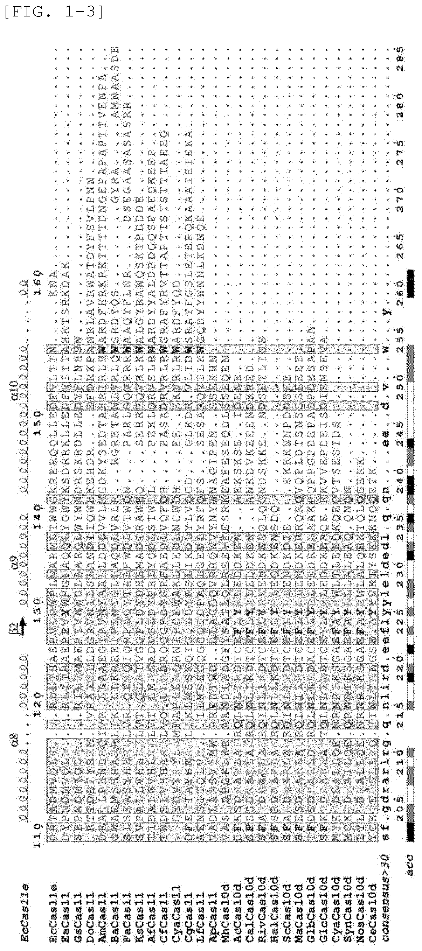

FIG. 1-1 shows comparisons between various types of ID Cas10d C-ter and Cas11e by alignment analysis.
FIG. 1-2 shows comparisons between various types of ID Cas10d C-ter and Cas11e by alignment analysis (continued from FIG. 1-1).
FIG. 1-3 shows comparisons between various types of ID Cas10d C-ter and Cas11e by alignment analysis (continued from FIG. 1-2).
FIG.2 shows effects of Cas10d C-ter protein overexpression in animal cells on genome editing activity.

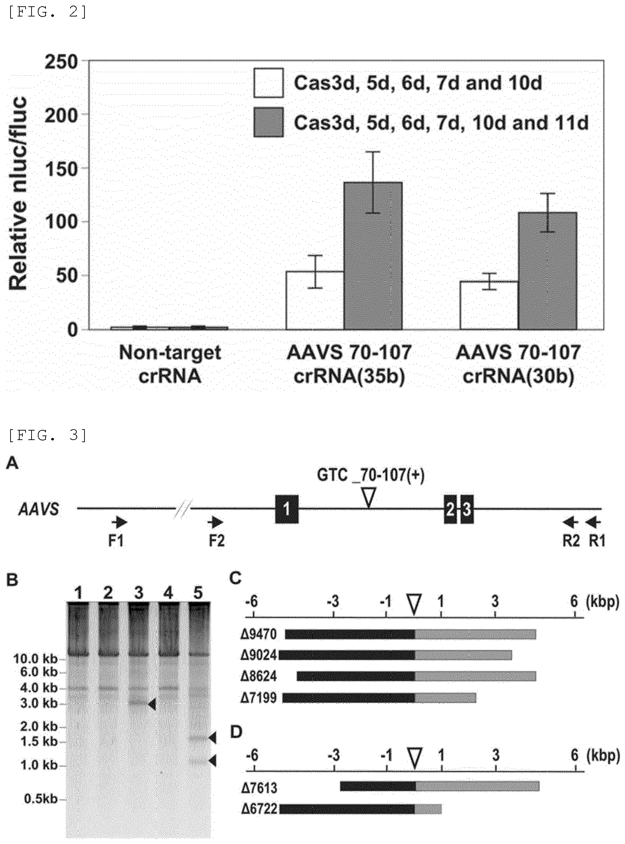

FIG.3 shows detection of long-range deletion mutations in the AAVS gene induced by the CRISPR TiD. A) Human AAVS gene structure, a gRNA position (open triangle), and different primer sets for amplifying the mutations (black arrows) are indicated. B) PCR amplified fragments separated on agarose gels are shown. Filled triangles indicate PCR products derived from the long-range deletions. C) Long-range deletion patterns induced by TiD comprising a Cas11d expression vector are shown. Black bars indicate deletion of 5' upstream ranges from the target sequence. Gray bars indicate deletion of 3' downstream ranges from the target sequence. Numbers at the left side of the bars indicate total lengths of deleted bases. D) Long-range deletion patterns induced by TiD not comprising a Cas11d expression vector are shown. Black bars indicate deletion of 5' upstream ranges from the target sequence. Gray bars indicate deletion of 3' downstream ranges from the target sequence. Numbers at the left side of the bars indicate total lengths of deleted bases.

### Mode for Carrying Out the Invention

The TiD system specifically comprises, among CRISPR type I-D Cas proteins, Cas3d, Cas5d, Cas6d, Cas7d and Cas10d as Cas effector proteins, and a TiD crRNA. It has been found that in the TiD system, a target recognition module (Cascade) is composed of Cas5d, Cas6d and Cas7d, and a polynucleotide cleavage module is composed of Cas3d and Cas10d (see Patent Literature 1). Further, the previous investigation by the present inventors has revealed that, of the elements constituting the cleavage module, Cas10d has polynucleotide degradation activity (nuclease activity) and Cas3d does not have nuclease activity. Specifically, in the TiD system, the TiD crRNA and the target recognition module target a target nucleotide sequence to guide the polynucleotide cleavage module to the vicinity of the target nucleotide sequence, and then, the target nucleotide sequence is cleaved by the action of Cas10d. In the TiD system, the TiD crRNA comprises a sequence capable of forming a base pair with a target nucleotide sequence (e.g., a sequence complementary to a target nucleotide sequence).

The present invention provides a method for targeting a target nucleotide sequence (hereinafter also referred to as "the target sequence-targeting method of the present invention"), a method for altering a target nucleotide sequence (hereinafter referred to as "the target sequence-altering method of the present invention"), and a method for regulating the expression of a target gene (hereinafter also referred to as "the target gene expression-regulating method of the present invention"), wherein the TiD system is utilized in the methods. Furthermore, the present invention provides a complex comprising CRISPR type I-D-associated Cas proteins and a crRNA (hereinafter also referred to as "the complex of the present invention"), a vector comprising a nucleic acid molecule encoding the complex (hereinafter also referred to as "the vector of the present invention"), and a kit (hereinafter also referred to as "the kit of the present invention"), which are used in the above-mentioned methods of the present invention.

The present invention is particularly characterized by using a polypeptide comprising a C-terminal partial sequence of Cas10d in addition to the above-mentioned Cas proteins in the TiD system. Interestingly, it was found that a common alpha-helix region is conserved between the C-terminal sequence of Cas10d and Cas11e (see Example 1). Thus the C-terminus of Cas10d was expected to fulfill the function of Cas11e, and thereby, to have no need of the expression of Cas11e unlike CRISPR type I-E. Surprisingly, however, it was found that the effect of the TiD system is increased by expressing a polypeptide containing a C-terminal partial sequence of Cas10d. Therefore, the present invention further provides a method for improving the efficiency of targeting or altering a target nucleotide sequence by the TiD system, comprising using a polypeptide comprising a C-terminal partial sequence of Cas10d, and a composition for improving the efficiency of targeting or altering a nucleotide sequence by the TiD system, comprising a polypeptide comprising a C-terminal partial sequence of Cas10d.

### (1) Cell

In the present invention, the cell may be either a prokaryotic cell or a eukaryotic cell, and is not particularly limited. Examples of the cell include bacteria, archaea, eukaryotes (e.g., yeast, filamentous fungi), plant cells, insect cells, and animal cells (e.g., human cells, non-human animal cells, mammalian cells, non-mammalian vertebrate cells, invertebrate cells, etc.). Preferably, a eukaryotic cell is used. As used herein, the "cell" includes a cell isolated from a living body, a cell existing in a living body (e.g., an animal body or a plant body), a living body (e.g., an animal body, or plants), and a cultured cell. The method of the present invention may be applied to a cell isolated from a living body, a cell existing in a living body, or a cell derived from any organ or tissue of a living body. For example, the method of the present invention may be applied to a cell existing in the body of a non-human animal or a non-human animal body itself. For example, the animal cells include, but not limited to, germ cells, fertilized eggs, embryonic cells, stem cells (including iPS cells, embryonic stem cells, somatic stem cells, etc.), and somatic cells. For example, the plant cells include, but not limited to, germ cells, fertilized eggs, embryonic cells, and somatic cells. As the plant cells, protoplasts may also be used.

### (2) Cas effector protein and nucleic acid encoding the protein

The Cas effector proteins used in the present invention are, among TiD Cas proteins, Cas3d, Cas5d, Cas6d, Cas7d, and Cas10d. The Cas3d, Cas5d, Cas6d, Cas7d and Cas10d may be derived from any bacterium or archaeon. Examples of the bacterium and the archaeon include *Microcystis aeruginosa, Acetohalobium arabaticum, Ammonifex degensii, Anabaena cylindrica, Anabaena variabilis, Caldicellulosiruptor lactoaceticus, Caldilinea aerophila, Crinalium epipsammum, Cyanothece Sp., Cylindrospermum stagnale, Haloquadratum walsbyi, Halorubrum lacusprofundi, Methanocaldococcus vulcanius, Methanospirillum hungatei, Natrialba asiatica, Natronomonas pharaonis, Nostoc punctiforme, Phormidesmis priestleyi, Oscillatoria acuminata, Picrophilus torridus, Spirochaeta thermophila, Stanieria cyanosphaera, Sulfolobus acidocaldarius, Sulfolobus islandicus, Synechocystis Sp., Thermacetogenium phaeum, Thermofilum pendens, Calothrix parietina, Gloeothece citriformis, Gloeobacter kilaueensis, Gloeocapsa sp., Halothece sp., Nostoc sp., Rivularia sp.* etc. In the present invention, Cas3d, Cas5d, Cas6d, Cas7d, and Cas10d may be derived from two or more bacterial or archaeal species, or may be derived from the same bacterial or archaeal species. Preferably, Cas proteins derived from the same bacterial or archaeal species are used. The amino acid sequence information and nucleotide sequence information of the Cas proteins are available from public database, for example, NCBI GenBank. In addition, the sequences from novel microbial species can be also obtained from microbial genome data obtained by metagenomic analysis or the like by using the BLAST program.

The Cas proteins can be obtained by known methods. For example, the Cas proteins may be chemically synthesized based on the amino acid sequence information, or produced in a cell by introducing nucleic acids encoding the Cas proteins into the cell via an appropriate vector or the like. The nucleic acids encoding the Cas proteins can be obtained by known methods. For example, the nucleic acids encoding the Cas proteins may be constructed by chemical synthesis or the like after selecting optimum codons for translation in a host cell into which the nucleic acids are introduced on the basis of the amino acid sequence information. Use of codons that are frequently used in the host cell makes it possible to increase the expression level of proteins. Examples of the nucleic acid include RNA such as mRNA, and DNA.

Cas10d is known to have an HD (histidine-aspartic acid) domain in the N-terminal region, in which the HD domain functions for DNA cleavage (see Patent Literature 2). In the present invention, it was further found that plural α-helix regions exist in the C-terminal region of Cas10d (Example 1). Therefore, Cas10d used in the present invention may be a polypeptide containing at least the N-terminal HD domain. For example, the Cas10d may be the full-length Cas10d protein, a polypeptide containing a region extending from the N-terminal HD domain to one or more C-terminal α-helix regions of Cas10d, or a polypeptide containing the N-terminal HD domain of Cas10d and lacking one or more C-terminal α-helix regions of Cas10d. The Cas10d may lack all of the C-terminal α-helix regions. As used herein, the term "Cas10d" includes the full-length Cas10d polypeptide and Cas10d fragments containing the N-terminal HD domain as described above.

Each Cas protein of Cas3d, Cas5d, Cas6d, Cas7d and Cas10d or a nucleic acid encoding each Cas protein may comprise one or more (for example one to several) amino acid mutations or one or more (for example one to several) nucleotide mutations, as long as a complex of the Cas proteins with a crRNA can target or alter a target sequence. As used herein, the term "several" refers to about 2 to 10, for example, 3, 4, 5, 6, 7, 8 or 9. As used herein, the term "mutation" includes deletion, substitution, insertion and addition of an amino acid or a nucleotide as compared to the native sequence.

Examples of the Cas proteins include, but not limited to, Cas3d from *Microcystis aeruginosa* (hereinafter referred to as *M. aeruginosa*) (SEQ ID NO: 1), Cas5d from *M. aeruginosa* (SEQ ID NO: 2), Cas6d from *M. aeruginosa* (SEQ ID NO: 3), Cas7d from *M. aeruginosa* (SEQ ID NO: 4), and Cas10d from *M. aeruginosa* (SEQ ID NO: 5). Therefore, an example of Cas3d used in the present invention is a protein comprising an amino acid sequence shown in SEQ ID NO: 1. An example of Cas5d used in the present invention is a protein comprising an amino acid sequence shown in SEQ ID NO: 2. An example of Cas6d used in the present invention is a protein comprising an amino acid sequence shown in SEQ ID NO: 3. An example of Cas7d used in the present invention is a protein comprising an amino acid sequence shown in SEQ ID NO:4. An example of Cas10d used in the present invention is a protein comprising an amino acid sequence shown in SEQ ID NO:5. A preferable example of Cas3d used in the present invention is a protein consisting of an amino acid sequence shown in SEQ ID NO: 1. A preferable example of Cas5d used in the present invention is a protein consisting of an amino acid sequence shown in SEQ ID NO: 2. A preferable example of Cas6d used in the present invention is a protein consisting of an amino acid sequence shown in SEQ ID NO: 3. A preferable example of Cas7d used in the present invention is a protein consisting of an amino acid sequence shown in SEQ ID NO: 4. A preferable example of Cas10d used in the present invention is a protein consisting of an amino acid sequence shown in SEQ ID NO: 5.

Further examples of the Cas proteins used in the present invention include proteins comprising amino acid sequences having 30% or more, 40% or more, 50% or more, 60% or more, 70% or more, or 80% or more, preferably 90% or more, further preferably 95% or more, still further preferably 96% or more, even further preferably 97% or more, more further preferably 98% or more, still more further preferably 99% or more sequence identity with SEQ ID NO: 1, SEQ ID NO: 2, SEQ ID NO: 3, SEQ ID NO: 4, or SEQ ID NO: 5. Preferably, further examples of the Cas proteins used in the present invention include proteins consisting of amino acid sequences having 30% or more, 40% or more, 50% or more, 60% or more, 70% or more, or 80% or more, preferably 90% or more, further preferably 95% or more, still further preferably 96% or more, even further preferably 97% or more, more further preferably 98% or more, still more further preferably 99% or more sequence identity with SEQ ID NO: 1, SEQ ID NO: 2, SEQ ID NO: 3, SEQ ID NO: 4, or SEQ ID NO: 5. Any of the Cas proteins as described above are capable of targeting or altering target sequences when complexed with the other Cas proteins and a crRNA.

When the cell into which the TiD system is introduced is a eukaryotic cell, a nuclear localizing signal sequence may be preferably added to the terminus of the Cas protein. The nuclear localizing signal sequence is known in the art, and can be appropriately selected depending on organism species that the cell into which the TiD system is introduced is derived from. Two or more nuclear localizing signal sequences may be tandemly arranged and added to the Cas protein. The nuclear localizing signal sequence may be added to either the N-terminus or the C-terminus of the Cas protein or both the N-terminus and the C-terminus of the Cas protein.

An example of a nucleic acid encoding Cas3d used in the present invention is a nucleic acid comprising a nucleotide sequence encoding a protein comprising an amino acid sequence shown in SEQ ID NO: 1. An example of a nucleic acid encoding Cas5d used in the present invention is a nucleic acid comprising a nucleotide sequence encoding a protein comprising an amino acid sequence shown in SEQ ID NO: 2. An example of a nucleic acid encoding Cas6d used in the present invention is a nucleic acid comprising a nucleotide sequence encoding a protein comprising an amino acid sequence shown in SEQ ID NO: 3. An example of a nucleic acid encoding Cas7d used in the present invention is a nucleic acid comprising a nucleotide sequence encoding a protein comprising an amino acid sequence shown in SEQ ID NO: 4. An example of a nucleic acid encoding Cas10d used in the present invention is a nucleic acid comprising a nucleotide sequence encoding a protein comprising an amino acid sequence shown in SEQ ID NO: 5. A preferable example of a nucleic acid encoding Cas3d used in the present invention is a nucleic acid comprising a nucleotide sequence encoding a protein consisting of an amino acid sequence shown in SEQ ID NO: 1. A preferable example of a nucleic acid encoding Cas5d used in the present invention is a nucleic acid comprising a nucleotide sequence encoding a protein consisting of an amino acid sequence shown in SEQ ID NO: 2. A preferable example of a nucleic acid encoding Cas6d used in the present invention is a nucleic acid comprising a nucleotide sequence encoding a protein consisting of an amino acid sequence shown in SEQ ID NO: 3. A preferable example of a nucleic acid encoding Cas7d used in the present invention is a nucleic acid comprising a nucleotide sequence encoding a protein consisting of an amino acid sequence shown in SEQ ID NO: 4. A preferable example of a nucleic acid encoding Cas10d used in the present invention is a nucleic acid comprising a nucleotide sequence encoding a protein consisting of an amino acid sequence shown in SEQ ID NO: 5. A further preferable example of a nucleic acid encoding Cas3d used in the present invention is a nucleic acid consisting of a nucleotide sequence encoding a protein consisting of an amino acid sequence shown in SEQ ID NO: 1. A further preferable example of a nucleic acid encoding Cas5d used in the present invention is a nucleic acid consisting of a nucleotide sequence encoding a protein consisting of an amino acid sequence shown in SEQ ID NO: 2. A further preferable example of a nucleic acid encoding Cas6d used in the present invention is a nucleic acid consisting of a nucleotide sequence encoding a protein consisting of an amino acid sequence shown in SEQ ID NO: 3. A further preferable example of a nucleic acid encoding Cas7d used in the present invention is a nucleic acid consisting of a nucleotide sequence encoding a protein consisting of an amino acid sequence shown in SEQ ID NO: 4. A further preferable example of a nucleic acid encoding Cas10d used in the present invention is a nucleic acid consisting of a nucleotide sequence encoding a protein consisting of an amino acid sequence shown in SEQ ID NO: 5.

Further examples of nucleic acids encoding the Cas proteins used in the present invention include nucleic acids comprising nucleotide sequences encoding proteins comprising amino acid sequences having 30% or more, 40% or more, 50% or more, 60% or more, 70% or more, or 80% or more, preferably 90% or more, further preferably 95% or more, still further preferably 96% or more, even further preferably 97% or more, more further preferably 98% or more, still more further preferably 99% or more sequence identity with SEQ ID NO: 1, SEQ ID NO: 2, SEQ ID NO: 3, SEQ ID NO: 4, or SEQ ID NO: 5. Preferably, further examples of nucleic acids encoding the Cas proteins used in the present invention include nucleic acids comprising nucleotide sequences encoding proteins consisting of amino acid sequences having 30% or more, 40% or more, 50% or more, 60% or more, 70% or more, or 80% or more, preferably 90% or more, further preferably 95% or more, still further preferably 96% or more, even further preferably 97% or more, more further preferably 98% or more, still more further preferably 99% or more sequence identity with SEQ ID NO: 1, SEQ ID NO: 2, SEQ ID NO: 3, SEQ ID NO: 4, or SEQ ID NO: 5. Further preferably, further examples of nucleic acids encoding the Cas proteins used in the present invention include nucleic acids consisting of nucleotide sequences encoding proteins consisting of amino acid sequences having 30% or more, 40% or more, 50% or more, 60% or more, 70% or more, or 80% or more, preferably 90% or more, further preferably 95% or more, still further preferably 96% or more, even further preferably 97% or more, more further preferably 98% or more, still more further preferably 99% or more sequence identity with SEQ ID NO: 1, SEQ ID NO: 2, SEQ ID NO: 3, SEQ ID NO: 4, or SEQ ID NO: 5. The Cas proteins expressed from any of the nucleic acids as described above are capable of targeting or altering target sequences when complexed with the Cas proteins expressed from the other nucleic acids and a crRNA.

When the cell into which the TiD system is introduced is a eukaryotic cell, a nucleotide sequence encoding a nuclear localizing signal may be preferably added to the terminus of the nucleic acid encoding the Cas protein. The nuclear localizing signal sequence is known in the art, and can be appropriately selected depending on organism species that the cell into which the TiD system is introduced is derived from. Two or more nuclear localizing signal sequences may be tandemly arranged and added to the nucleic acid encoding the Cas protein. The nuclear localizing signal sequence may be added to either the 5' end or the 3' end of the nucleic acid encoding the Cas protein or both the 5' end and the 3' end of the nucleic acid encoding the Cas protein.

### (3) Polypeptide encoding C-terminal partial sequence of Cas10d (Cas10d C-ter) and nucleic acid encoding the polypeptide

In the present invention, the C-terminal partial sequence of Cas10d (Cas10d C-ter) does not contain the N-terminal HD domain of Cas10d and contains one or more C-terminal α-helix regions of Cas10d. In the present invention, a polypeptide containing Cas10d C-ter (hereinafter also referred to as "Cas11d") does not contain the N-terminal HD domain of Cas10d.

The length of Cas10d C-ter is not particularly limited as long as the effect of the present invention is achieved, that is, as long as efficient targeting and alteration of a target nucleotide sequence by the TiD system is achieved. For example, Cas10d C-ter may be about 100 amino acids to about 400 amino acids in length, preferably about 120 amino acids to about 270 amino acids in length, more preferably about 130 amino acids to about 180 amino acids in length, even more preferably about 135 amino acids to about 170 amino acids in length. Examples of Cas10d C-ter include polypeptides of about 100 to about 400 amino acids in length, preferably about 120 to about 270 amino acids in length, more preferably about 130 to about 180 amino acids in length, even more preferably about 135 to about 170 amino acids in length from the C-terminus of the full length amino acid sequence of Cas10d. Examples of nucleic acids encoding Cas10d C-ter include polynucleotides of about 0.3 kb to about 1.2 kb in length, preferably about 0.36 kb to about 0.81 kb in length, more preferably about 0.39 kb to about 0.54 kb in length, even more preferably about 0.41 kb to about 0.51 kb in length 5' upstream from the stop codon of the Cas10d gene. Thus, Cas11d may comprise, for example, about 100 amino acids to about 400 amino acids, preferably about 120 amino acids to about 270 amino acids, more preferably about 130 amino acids to about 180 amino acids, more preferably about 135 amino acids to about 170 amino acids from the C-terminus of the full-length amino acid sequence of Cas10d. Preferable examples of Cas11d include polypeptides consisting of about 100 amino acids to about 400 amino acids, preferably about 120 amino acids to about 270 amino acids, more preferably about 130 amino acids to about 180 amino acids, more preferably about 135 amino acids to about 170 amino acids from the C-terminus of the full-length amino acid sequence of Cas10d. The nucleic acid encoding Cas11d may comprise, for example, about 0.3 kb to about 1.2 kb, preferably about 0.36 kb to about 0.81 kb, more preferably about 0.39 kb to about 0.54 kb, even more preferably about 0.41 kb to about 0.51 kb 5' upstream from the stop codon of the Cas10d gene. Preferable examples of the nucleic acid encoding Cas11d include nucleic acids consisting of about 0.3 kb to about 1.2 kb, preferably about 0.36 kb to about 0.81 kb, more preferably about 0.39 kb to about 0.54 kb, even more preferably about 0.41 kb to about 0.51 kb 5' upstream from the stop codon of the Cas10d gene. However, Cas11d is not full-length Cas10d.

The Cas11d and the nucleic acid encoding Cas11d can be obtained by known methods. For example, the Cas11d may be chemically synthesized based on the amino acid sequence information, or produced in a cell by introducing the nucleic acid encoding the Cas11d into the cell via an appropriate vector or the like. The nucleic acid encoding the Cas11d may be constructed for example by chemical synthesis or the like after selecting optimum codons for translation in a host cell into which the nucleic acid is introduced on the basis of the amino acid sequence information. Use of codons that are frequently used in the host cell makes it possible to increase the expression level of protein. Examples of the nucleic acid include RNA such as mRNA, and DNA.

The Cas11d or the nucleic acid encoding Cas11d may comprise one or more (for example one to several) amino acid mutations or one or more (for example one to several) nucleotide mutations, as long as the effect of the present invention is achieved, that is, as long as a complex of the Cas11d and the Cas proteins as described above with a crRNA can induce efficient targeting and alteration of a target nucleotide sequence.

Examples of the Cas11d include, but not limited to, polypeptides comprising Cas10d C-ter from *M. aeruginosa* Cas10d (SEQ ID NO: 5). Therefore, examples of Cas11d used in the present invention include polypeptides comprising about 100 amino acids to about 400 amino acids, preferably about 120 amino acids to about 270 amino acids, more preferably about 130 amino acids to about 180 amino acids, more preferably about 135 amino acids to about 170 amino acids from the C-terminus of the amino acid sequence shown by SEQ ID NO: 5. Preferable examples of Cas11d used in the present invention include polypeptides consisting of about 100 amino acids to about 400 amino acids, preferably about 120 amino acids to about 270 amino acids, more preferably about 130 amino acids to about 180 amino acids, more preferably about 135 amino acids to about 170 amino acids from the C-terminus of the amino acid sequence shown by SEQ ID NO: 5. An example of Cas11d derived from *M. aeruginosa* is a polypeptide comprising a sequence (SEQ ID NO: 6) consisting of amino acids at positions 997 to 1156 in the amino acid sequence shown by SEQ ID NO: 5. A preferable example of Cas11d derived from *M. aeruginosa* is a polypeptide consisting of the amino acid sequence shown by SEQ ID NO: 6.

Further, examples of Cas11d derived from *Anabaena cylindrica, Calothrix* PCC6303 (*Calothrix parietina*), *Crinalium epipsammum, Cyanothece* PCC7424 (*Gloeothece citriformis*), *Gloeobacter kilaueensis, Gloeocapsa sp.* PCC7428, *Halothece* PCC7418, *Methanospirillum hungatei, Nostoc sp.* NIES-2111, *Rivularia sp.* PCC7116, *Stanieria cyanosphaera,* and *Synechocystis sp.* PCC6803 include polypeptides comprising amino acid sequences shown by SEQ ID NOs: 8 to 19, respectively. Further preferable examples thereof include polypeptides consisting of amino acid sequences shown by SEQ ID NOs: 8 to 19. In the present invention, Cas11d may comprise Cas10d C-ter derived form a different bacterial or archaeal species from or the same bacterial or archaeal as those of the above-described Cas proteins (Cas3d, Cas5d, Cas6d, Cas7d, and/or Cas10d). Preferably, a polypeptide comprising Cas10d C-ter that is derived from the same bacterial or archaeal species as any of the above-described Cas proteins is used as Cas11d.

Further examples of Cas11d used in the present invention include polypeptides comprising amino acid sequences having 30% or more, 40% or more, 50% or more, 60% or more, 70% or more, or 80% or more, preferably 90% or more, further preferably 95% or more, still further preferably 96% or more, even further preferably 97% or more, more further preferably 98% or more, still more further preferably 99% or more sequence identity with polypeptides consisting of about 100 amino acids to about 400 amino acids, preferably about 120 amino acids to about 270 amino acids, more preferably about 130 amino acids to about 180 amino acids, more preferably about 135 amino acids to about 170 amino acids from the C-terminus of the amino acid sequence shown by SEQ ID NO: 5. Further preferable examples of Cas11d used in the present invention include polypeptides consisting of amino acid sequences having 30% or more, 40% or more, 50% or more, 60% or more, 70% or more, or 80% or more, preferably 90% or more, further preferably 95% or more, still further preferably 96% or more, even further preferably 97% or more, more further preferably 98% or more, still more further preferably 99% or more sequence identity with polypeptides consisting of about 100 amino acids to about 400 amino acids, preferably about 120 amino acids to about 270 amino acids, more preferably about 130 amino acids to about 180 amino acids, more preferably about 135 amino acids to about 170 amino acids from the C-terminus of the amino acid sequence shown by SEQ ID NO: 5. Further examples of Cas11d include polypeptides comprising amino acid sequences having 30% or more, 40% or more, 50% or more, 60% or more, 70% or more, or 80% or more, preferably 90% or more, further preferably 95% or more, still further preferably 96% or more, even further preferably 97% or more, more further preferably 98% or more, still more further preferably 99% or more sequence identity with the amino acid sequence shown by any of SEQ ID NO: 6 and SEQ ID NOs: 8 to 19. Preferable examples of Cas11d include polypeptides consisting of amino acid sequences having 30% or more, 40% or more, 50% or more, 60% or more, 70% or more, or 80% or more, preferably 90% or more, further preferably 95% or more, still further preferably 96% or more, even further preferably 97% or more, more further preferably 98% or more, still more further preferably 99% or more sequence identity with the amino acid sequence shown by any of SEQ ID NO: 6 and SEQ ID NOs: 8 to 19. Any Cas11d as described above is capable of inducing efficient targeting or alteration of a target sequence when complexed with the above-described Cas proteins and a crRNA.

When the cell into which the TiD system is introduced is a eukaryotic cell, a nuclear localizing signal sequence may be preferably added to the terminus of Cas11d. The nuclear localizing signal sequence is known in the art, and can be appropriately selected depending on organism species that the cell into which the TiD system is introduced is derived from. Two or more nuclear localizing signal sequences may be tandemly arranged and added to Cas11d. The nuclear localizing signal sequence may be added to either the N-terminus or the C-terminus of Cas11d or both the N-terminus and the C-terminus of Cas11d.

Examples of the nucleic acid encoding Cas11d used in the present invention include nucleic acids comprising about 0.3 kb to about 1.2 kb, preferably about 0.36 kb to about 0.81 kb, more preferably about 0.39 kb to about 0.54 kb, even more preferably about 0.41 kb to about 0.51 kb 5' upstream from the stop codon in a nucleotide sequence encoding a protein comprising the amino acid sequence shown by SEQ ID NO: 5. Preferable examples of the nucleic acid encoding Cas11d used in the present invention include nucleic acids comprising about 0.3 kb to about 1.2 kb, preferably about 0.36 kb to about 0.81 kb, more preferably about 0.39 kb to about 0.54 kb, even more preferably about 0.41 kb to about 0.51 kb 5' upstream from the stop codon in a nucleotide sequence encoding a protein consisting of the amino acid sequence shown by SEQ ID NO: 5. Further preferable examples of the nucleic acid encoding Cas11d used in the present invention include nucleic acids consisting of about 0.3 kb to about 1.2 kb, preferably about 0.36 kb to about 0.81 kb, more preferably about 0.39 kb to about 0.54 kb, even more preferably about 0.41 kb to about 0.51 kb 5' upstream from the stop codon in a nucleotide sequence encoding a protein consisting of the amino acid sequence shown by SEQ ID NO: 5. Examples of the nucleic acid encoding Cas11d include a nucleic acid comprising a nucleotide sequence encoding a polypeptide comprising the amino acid sequence shown by SEQ ID NO: 6, preferably a nucleic acid comprising a nucleotide sequence encoding a polypeptide consisting of the amino acid sequence shown by SEQ ID NO: 6, and further preferably a nucleic acid consisting of a nucleotide sequence encoding a polypeptide consisting of the amino acid sequence shown by SEQ ID NO: 6. Other examples of the nucleic acid encoding Cas11d include nucleic acids comprising nucleotide sequences encoding polypeptides comprising the amino acid sequences shown by SEQ ID NOs: 8 to 19, preferably nucleic acids comprising nucleotide sequences encoding polypeptides consisting of the amino acid sequences shown by SEQ ID NOs: 8 to 19, and further preferably nucleic acids consisting of nucleotide sequences encoding polypeptides consisting of the amino acid sequences shown by SEQ ID NOs: 8 to 19.

Further examples of the nucleic acid encoding Cas11d used in the present invention include nucleic acids comprising nucleotide sequences having 30% or more, 40% or more, 50% or more, 60% or more, 70% or more, or 80% or more, preferably 90% or more, further preferably 95% or more, still further preferably 96% or more, even further preferably 97% or more, more further preferably 98% or more, still more further preferably 99% or more sequence identity with a sequence consisting of about 0.3 kb to about 1.2 kb, preferably about 0.36 kb to about 0.81 kb, more preferably about 0.39 kb to about 0.54 kb, even more preferably about 0.41 kb to about 0.51 kb 5' upstream from the stop codon in a nucleotide sequence encoding a protein comprising the amino acid sequence shown by SEQ ID NO: 5. Preferable examples of the nucleic acid encoding Cas11d used in the present invention include nucleic acids comprising nucleotide sequences having 30% or more, 40% or more, 50% or more, 60% or more, 70% or more, or 80% or more, preferably 90% or more, further preferably 95% or more, still further preferably 96% or more, even further preferably 97% or more, more further preferably 98% or more, still more further preferably 99% or more sequence identity with a sequence consisting of about 0.3 kb to about 1.2 kb, preferably about 0.36 kb to about 0.81 kb, more preferably about 0.39 kb to about 0.54 kb, even more preferably about 0.41 kb to about 0.51 kb 5' upstream from the stop codon in a nucleotide sequence encoding a protein consisting of the amino acid sequence shown by SEQ ID NO: 5. Further preferable examples of the nucleic acid encoding Cas11d used in the present invention include nucleic acids consisting of nucleotide sequences having 30% or more, 40% or more, 50% or more, 60% or more, 70% or more, or 80% or more, preferably 90% or more, further preferably 95% or more, still further preferably 96% or more, even further preferably 97% or more, more further preferably 98% or more, still more further preferably 99% or more sequence identity with a sequence consisting of about 0.3 kb to about 1.2 kb, preferably about 0.36 kb to about 0.81 kb, more preferably about 0.39 kb to about 0.54 kb, even more preferably about 0.41 kb to about 0.51 kb 5' upstream from the stop codon in a nucleotide sequence encoding a protein consisting of the amino acid sequence shown by SEQ ID NO: 5. Other examples of the nucleic acid encoding Cas11d include nucleic acids comprising nucleotide sequences having 30% or more, 40% or more, 50% or more, 60% or more, 70% or more, or 80% or more, preferably 90% or more, further preferably 95% or more, still further preferably 96% or more, even further preferably 97% or more, more further preferably 98% or more, still more further preferably 99% or more sequence identity with nucleotide sequences encoding polypeptides comprising the amino acid sequences shown by any of SEQ ID NO: 6 and SEQ ID NOs: 8 to 19. Further other examples of the nucleic acid encoding Cas11d include nucleic acids comprising nucleotide sequences having 30% or more, 40% or more, 50% or more, 60% or more, 70% or more, or 80% or more, preferably 90% or more, further preferably 95% or more, still further preferably 96% or more, even further preferably 97% or more, more further preferably 98% or more, still more further preferably 99% or more sequence identity with nucleotide sequences encoding polypeptides consisting of the amino acid sequences shown by any of SEQ ID NO: 6 and SEQ ID NOs: 8 to 19. Further other examples of the nucleic acid encoding Cas11d include nucleic acids consisting of nucleotide sequences having 30% or more, 40% or more, 50% or more, 60% or more, 70% or more, or 80% or more, preferably 90% or more, further preferably 95% or more, still further preferably 96% or more, even further preferably 97% or more, more further preferably 98% or more, still more further preferably 99% or more sequence identity with nucleotide sequences encoding polypeptides consisting of the amino acid sequences shown by any of SEQ ID NO: 6 and SEQ ID NOs: 8 to 19. The Cas11d polypeptide expressed from any of the nucleic acids as described above is capable of inducing efficient targeting or alteration of a target sequence when complexed with the above-described Cas proteins and a crRNA.

When the cell into which the TiD system is introduced is a eukaryotic cell, a nucleotide sequence encoding a nuclear localizing signal may be preferably added to the terminus of the nucleic acid encoding Cas11d. The nuclear localizing signal sequence is known in the art, and can be appropriately selected depending on organism species that the cell into which the TiD system is introduced is derived from. Two or more nuclear localizing signal sequences may be tandemly arranged and added to the nucleic acid encoding Cas11d. The nuclear localizing signal sequence may be added to either the 5' end or the 3' end of the nucleic acid encoding Cas11d or both the 5' end and the 3' end of the nucleic acid encoding Cas11d.

### (4) crRNA

The crRNA comprises one or more structural units ("repeat-spacer-repeat") consisting of repeat sequences derived from a CRISPR locus and a spacer sequence sandwiched between the repeat sequences. The repeat sequences preferably contain palindrome-like sequences. The crRNA contains, as the spacer sequence, an RNA sequence (i.e., protospacer sequence) capable of binding to a target nucleotide sequence, and thus contributes to the target recognition of CRISPR-Cas systems. An RNA molecule comprising a structure consisting of crRNA repeat sequences and a protospacer sequence sandwiched between the repeat sequences is also called a guide RNA (gRNA). The crRNA is processed by the action of Cas effector proteins to cleave the repeat sequences, and thereby a mature crRNA consisting of partial sequences of the repeat sequences and the protospacer sequence sandwiched between the partial sequences of the repeat sequences is obtained. The crRNA before being processed is called a pre-mature crRNA.

The crRNA used in the present invention comprises repeat sequences derived from the CRISPR type I-D locus, and a sequence capable of forming a base pair with a target nucleotide sequence as the protospacer sequence sandwiched between the repeat sequences. The crRNA used in the present invention is preferably a pre-mature crRNA.

The pre-mature crRNA undergoes processing by Cas6d to become a mature crRNA, and the mature crRNA is then incorporated into a Cascade (a complex of Cas5d, Cas6d and Cas7d) . When the pre-mature crRNA comprises two or more "repeat-spacer-repeat" structural units, the pre-mature crRNA may comprise two or more kinds of protospacer sequences. The pre-mature crRNA comprising two or more kinds of protospacer sequences generates two or more kinds of mature crRNAs, and these mature crRNAs are then incorporated into Cascades separately.

The protospacer sequence contained in the crRNA is a sequence capable of forming a base pair with a target nucleotide sequence. Examples of the "sequence capable of forming a base-pair with a target nucleotide sequence" include a sequence that is complementary to the target nucleotide sequence, and a sequence that is substantially complementary to the target nucleotide sequence. The term "substantially complementary" includes a sequence that is not completely complementary to the target sequence but capable of binding to the target sequence (forming a base pair with the target sequence). The sequence that is substantially complementary to a target nucleotide sequence may contain bases mismatched to the target sequence as long as it forms base pairs with the target sequence.

The repeat sequence parts of crRNA may have at least one hairpin structure. For example, the repeat sequence part placed at the 5' end side of the protospacer sequence may have a hairpin structure, and the repeat sequence part placed at the 3' end side of the protospacer sequence may be single-stranded. In the present invention, the crRNA preferably has a hairpin structure.

The repeat sequence derived from the CRISPR type I-D locus can be found from a crRNA gene sequence region adjacent to the type I-D gene group by using a tandem repeat search program. The repeat sequence derived from the CRISPR type I-D locus may be derived from any bacterium or archaeon, and may be derived from, for example, bacteria or archaea as above cited relating to the Cas effector proteins.

The nucleotide length of the repeat sequence contained in the crRNA is not particularly limited as long as the crRNA interacts with the Cascade to target a target nucleotide sequence. For example, each of the repeat sequences preceding and following the protospacer sequence may have a length of about 10 to 70 nucleotides, for example, a length of about 30 to 50 nucleotides, preferably a length of about 35 to 45 nucleotides.

The crRNA used in the present invention can contain a protospacer sequence consisting of about 10 to 70 nucleotides. The protospacer sequence contained in the crRNA is preferably a sequence consisting of 20 to 50 nucleotides, more preferably a sequence consisting of 25 to 45 nucleotides, more preferably a sequence consisting of 30 to 40 nucleotides, or, for example, a sequence consisting of 31 nucleotides, 32 nucleotides, 33 nucleotides, 34 nucleotides, 35 nucleotides, 36 nucleotides, 37 nucleotides, 38 nucleotides, or 39 nucleotides. The sequence specificity of target recognition by the crRNA is more greatly increased as the target sequence that can be targeted is longer. In addition, the Tm value of a base pair formed between the crRNA and the target sequence is higher and thus the stability of target recognition is more greatly increased as the target sequence that can be targeted is longer. Since the length of a sequence that can be targeted by a crRNA for RNA-guided endonucleases (e.g., Cas9 and Cpf1) used in the conventional genome editing techniques is about 20 to 24 nucleotide length, the present invention is excellent in the sequence specificity and the stability as compared with the conventional methods.

Examples of the crRNA used in the present invention include, but not limited to, a crRNA comprising crRNA repeat sequences from *M. aeruginosa.* An example thereof is a pre-mature crRNA comprising a sequence shown by GUUCCAAUUAAUCUUAAGCCCUAUUAGGGAUUGAAACNNNNNNNNNNNNNNNNNNNNNN NNNNNNNNNNNNNGUUCCAAUUAAUCUUAAGCCCUAUUAGGGAUUGAAAC (SEQ ID NO:7; N is any nucleotide constituting a sequence that forms a base pair with a target nucleotide sequence). In the crRNA sequence, the number of N may be varied within a range of 10 to 70, preferably 20 to 50, more preferably 25 to 45, and still more preferably 30 to 40.

The crRNA may be introduced into the cell as an RNA or as a DNA encoding the crRNA. The DNA encoding the crRNA may be contained, for example, in a vector or an expression cassette. The DNA sequence is preferably operably linked to a regulatory sequence such as a promoter or a terminator. The vector or the regulatory sequence can be appropriately selected, for example, depending on a host cell, etc. Examples of the regulatory sequence include, but not limited to, pol III promoters (e.g., SNR6, SNR52, SCR1, RPR1, U6, H1 promoter, etc.), pol II promoters and terminators (e.g., T6 sequence), and human U6 snRNA promoters.

The DNA encoding the crRNA may be contained together with any of nucleic acids encoding the Cas proteins and the Cas11d polypeptide in the same vector or expression cassette, or may be contained in a separate vector or expression cassette from the nucleic acids encoding the Cas proteins and the Cas11d polypeptide.

### (5) Target nucleotide sequence

In the present invention, the target nucleotide sequence (also referred to as "the target sequence", as used herein) is any nucleic acid sequence, and is not particularly limited as long as it is a sequence located in the vicinity of a protospacer adjacent motif (PAM) in the TiD system. The nucleic acid may be a nucleic acid in a living body or cell or a nucleic acid isolated from a living body or cell. The target nucleotide sequence may be a double-stranded DNA sequence, a single-stranded DNA sequence, or an RNA sequence. Examples of DNA include eukaryotic nuclear genomic DNA, mitochondrial DNA, plastid DNA, prokaryotic genomic DNA, phage DNA, and plasmid DNA. In the present invention, the target nucleotide sequence is preferably a DNA on the genome. Thus, on the sense strand of a target nucleic acid, a sequence located in the vicinity of the PAM sequence, preferably a sequence located in the vicinity of the 3'-downstream side of the PAM sequence, more preferably a sequence located adjacent to the 3'-downstream side of the PAM sequence is selected as the target nucleotide sequence. Further, on the antisense strand of a target nucleic acid, a sequence located in the vicinity of the PAM sequence, preferably a sequence located in the vicinity of the 5' side of the PAM sequence, more preferably a sequence located adjacent to the 5' side of the PAM sequence is selected as the target nucleotide sequence. As used herein, the phrase "in the vicinity of" includes both being adjacent to a place and being close to a place. As used herein, the "vicinity" includes both adjacency and neighborhood. Unless otherwise specified, description herein is based on the sense strand.

The PAM sequences used for target recognition of CRISPR systems vary depending on the types of CRISPR systems. For example, the PAM sequence in the TiD system derived from some species including *M. aeruginosa* is 5'-GTH-3' (H = A, C or T) on the sense strand of a target nucleic acid, and is 5'-HTG-3' (H = A, C or T) on the antisense strand of a target nucleic acid (see Patent Literature 1).

For example, the target nucleotide sequence may be a sequence located in the vicinity of the PAM sequence and present in an intron, a coding region, a non-coding region, or a control region of a target gene. The target gene may be any gene and optionally selected.

The length of the target nucleotide sequence is, for example, 10 to 70 nucleotides in length, preferably 20 to 50 nucleotides in length, more preferably 25 to 45 nucleotides in length, even more preferably 30 to 40 nucleotides in length.

### (6) Target sequence-targeting method of the present invention

The method of targeting a target sequence of the present invention is characterized by introducing Cas5d, Cas6d, Cas7d and Cas10d among TiD Cas effector proteins, a polypeptide containing Cas10d C-ter (Cas11d), and a crRNA into a cell. Specifically, the target sequence-targeting method of the present invention is characterized by introducing into the cell (i) Cas5d, Cas6d, Cas7d ands Cas10d, or nucleic acids encoding these proteins, (ii) a Cas11d polypeptide, or a nucleic acid encoding the polypeptide, and (iii) a crRNA containing a sequence capable of forming a base pair with the target nucleotide sequence, or a DNA encoding the crRNA. The target sequence-targeting method of the present invention may be performed in vitro, in vivo, or ex vivo. The target sequence-targeting method of the present invention can also be applied to a target nucleotide sequence on an isolated nucleic acid, and in such a case, the method comprises bringing the Cas proteins of (i), the Cas11d polypeptide of (ii) and the crRNA of (iii) into contact with the isolated nucleic acid comprising the target nucleotide sequence.

In the target sequence-targeting method of the present invention, the above-mentioned Cas proteins may be introduced into the cell or contacted with the isolated nucleic acid comprising the target nucleotide sequence, as an isolated complex comprising two or more, for example four, of Cas5d, Cas6d, Cas7d and Cas10d, or each of Cas5d, Cas6d, Cas7d and Cas10d may be introduced into the cell or contacted with the isolated nucleic acid comprising the target nucleotide sequence, as an isolated single protein. In the target sequence-targeting method of the present invention, the Cas proteins may be also introduced into the cell as nucleic acids encoding Cas proteins Cas5d, Cas6d, Cas7d and Cas10d. Examples of the nucleic acid include RNA such as mRNA and DNA.

In the target sequence-targeting method of the present invention, Cas11d may be introduced into the cell or contacted with the isolated nucleic acid comprising the target nucleotide sequence, as an isolated complex comprising Cas11d and the above-mentioned Cas proteins, or Cas11d may be introduced into the cell or contacted with the isolated nucleic acid comprising the target nucleotide sequence, as an isolated single protein. In the target sequence-targeting method of the present invention, Cas11d may be also introduced into the cell as a nucleic acid encoding Cas11d. Examples of the nucleic acid include RNA such as mRNA and DNA.

The nucleic acids encoding the Cas proteins and Cas11d may be contained in, for example, a vector. The nucleic acid DNA sequence is preferably operably linked to a regulatory sequence such as a promoter or terminator. When the cell into which the Cas proteins Cas5d, Cas6d, Cas7d and Cas10d, and Cas11d are introduced is a eukaryotic cell, a nuclear localizing signal sequence is preferably added to the nucleic acid sequences encoding the Cas proteins and Cas11d. Two or more or all of the nucleic acids encoding the Cas proteins Cas5d, Cas6d, Cas7d and Cas10d, and Cas11d may be contained in a single vector or expression cassette, or may be contained in separate vectors or expression cassettes. The number of the vectors or expression cassettes, and the kinds and combinations of the nucleic acids which are incorporated into each vector or expression cassette are not limited. When two or more nucleic acids encoding the Cas proteins and Cas11d are contained in a single vector or expression cassette, the nucleic acid sequences may be linked to each other, for example via a sequence encoding a self-cleaving peptide, so as to be polycistronically expressed. The two or more nucleic acids encoding the Cas proteins and Cas11d may be linked in any order.

The crRNA may be introduced into the cell as an RNA or as a DNA encoding the crRNA. The crRNA may also be introduced into the cell or contacted with the isolated nucleic acid comprising the target nucleotide sequence, as a complex with the Cas proteins and/or Cas11d. The crRNA or the DNA encoding the crRNA may be contained, for example, in a vector. The RNA or DNA sequence is preferably operably linked to a regulatory sequence such as a promoter or a terminator. The crRNA or the DNA encoding the crRNA may be contained together with the nucleic acids encoding the Cas proteins and/or the nucleic acid encoding Cas11d in the same vector or expression cassette, or may be contained in a separate vector or expression cassette from the nucleic acids encoding the Cas proteins and/or the nucleic acid encoding Cas11d.

The vector is an expression vector for carrying a nucleic acid encoding a protein of interest into a desired cell to express the protein of interest in the cell. The expression cassette means a nucleic acid molecule that directs the transcription and/or translation of a nucleic acid encoding a protein of interest to allow the expression of the protein of interest. The expression cassette may be contained in the vector. Various kinds of vectors commonly used in the art can be used, and can be appropriately selected depending on the types of cells into which the vectors are introduced or the introduction methods. Examples of the vectors include, but not limited to, plasmid vectors, viral vectors, retroviral vectors, phages, phagemids, cosmids, artificial/minichromosomes, and transposons.

Examples of the regulatory sequences include promoters, enhancers, terminators, internal ribosome entry sites (IRES), polyadenylation signals, poly U sequences, and translation enhancers. The regulatory sequences are not particularly limited, and can be appropriately selected by those skilled in the art considering host cells and the like. For example, when the host is a plant cell, examples of the promoter include CaMV35S promoter, 2xCaMV35S promoter, CaMV19S promoter, and NOS promoter. When the host is an animal cell, examples of the promoter include SRα promoter, SV40 promoter, LTR promoter, CMV promoter, RSV promoter, MoMuLV LTR promoter, HSV-TS promoter, human translation elongation factor gene promoter, and CAG chimera synthetic promoter.

The nuclear localizing signal sequence is known in the art, and can be appropriately selected depending on organism species that the cells into which the Cas proteins, Cas11d and crRNA are introduced are derived from. For example, a monopartite nuclear localizing signals or a bipartite nuclear localizing signal may be used.

The introduction of the Cas proteins, Cas11d, and crRNA into the cell can be performed by various means known in the art. Examples of such means include transfection, e.g., calcium phosphate-mediated transfection, electroporation, liposome transfection, etc., virus transduction, lipofection, gene gun, microinjection, Agrobacterium method, Agroinfiltration, and a PEG-calcium method.

The Cas proteins, Cas11d, and crRNA may be introduced into the cell simultaneously or sequentially. The Cas proteins Cas5d, Cas6d, Cas7d and Cas10d, or nucleic acids encoding these Cas proteins may be introduced into the cell simultaneously or sequentially. For example, the Cas proteins Cas5d, Cas6d, Cas7d and Cas10d, and Cas11d that are synthesized in vitro or in vivo and the crRNA synthesized in vitro or in vivo may be incubated in vitro to form a complex, and the complex may be introduced into the cell.

Upon introduction of the Cas proteins, Cas11d, and crRNA, the cell is cultured under suitable conditions for targeting of a target nucleotide sequence. The cell is then cultured under suitable conditions for cell growth and maintenance. The culture conditions may be suitable for the organism species that the cell into which the Cas proteins, Cas11d and crRNA are introduced is derived from, and can be appropriately determined by a person skilled in the art, for example, based on known cell culture techniques.

In the target sequence-targeting method of the present invention, a fusion protein comprising the Cas proteins and a functional polypeptide may be used. In such a case, the fusion protein is guided to a target nucleotide sequence in the cell by the action of the Cas proteins and the crRNA, and the target nucleotide sequence is altered or modified by the action of the functional polypeptide. Thus the present invention further provides a method for altering or modifying a target nucleotide sequence, which comprises introducing the fusion protein, Cas11d and the crRNA into a cell or contacting the fusion protein, Cas11d and the crRNA with an isolated nucleic acid comprising the target nucleotide sequence. The functional polypeptide is a polypeptide that exhibits any function to a target sequence. Examples of the functional polypeptide include, but not limited to, restriction enzymes, transcription factors, DNA methylases, histone acetylases, fluorescent proteins; polynucleotide cleavage modules, for example, nucleotide cleavage modules of restriction enzymes; gene expression regulation modules, for example, transcription activation modules and transcription repression modules of transcription factors; and epigenomic modification modules, for example, methylation modules of DNA methylases, and histone acetylation modules of histone acetylases; and modules that induce base substitution, for example, cytosine deaminases, and adenine deaminases. Examples of the fluorescent protein include GFP.

According to the target sequence-targeting method of the present invention, the target sequence can be efficiently targeted due to the presence of Cas11d.

### (7) Target sequence-altering method of the present invention

The target sequence-altering method of the present invention is characterized by introducing Cas effector proteins Cas5d, Cas6d, Cas7d, Cas3d and Cas10d, a polypeptide containing Cas10d C-ter (Cas11d), and a crRNA into the cell. Specifically, the target sequence-altering method of the present invention is characterized by introducing into the cell (i) Cas3d, Cas5d, Cas6d, Cas7d and Cas10d, or nucleic acids encoding the proteins, (ii) a Cas11d polypeptide, or a nucleic acid encoding the polypeptide, and (iii) a crRNA containing a sequence capable of forming a base pair with the target nucleotide sequence, or a DNA encoding the crRNA. The target sequence-altering method of the present invention comprises cleaving a nucleotide sequence targeted by the target sequence-targeting method of the present invention, by the action of Cas3d and Cas10d. The target sequence-altering method of the present invention may be performed in vitro, in vivo, or ex vivo. In the present invention, the alteration includes deletion, insertion, and substitution of one or more nucleotides, and a combination thereof. The target sequence-altering method of the present invention can also be applied to a target nucleotide sequence on an isolated nucleic acid, and in such a case, the method comprises bringing the Cas proteins of (i), the Cas11d polypeptide of (ii) and the crRNA of (iii) into contact with the isolated nucleic acid comprising the target nucleotide sequence. When the target sequence-altering method of the present invention is applied to a target nucleotide sequence on an isolated nucleic acid, a method of cleaving the target nucleotide sequence is preferably provided.

In the target sequence-altering method of the present invention, Cas5d, Cas6d, Cas7d, Cas3d and Cas10d may be introduced into the cell or contacted with the isolated nucleic acid comprising the target nucleotide sequence, as an isolated complex comprising two or more of the five Cas proteins, for example an isolated complex comprising the five Cas proteins, or an isolated complex comprising Cas5d, Cas6d and Cas7d and/or an isolated complex comprising Cas3d and Cas10d, or each of Cas5d, Cas6d, Cas7d, Cas3d and Cas10d may be introduced into the cell or contacted with the isolated nucleic acid comprising the target nucleotide sequence, as an isolated single protein. The Cas proteins may be also introduced into the cell as nucleic acids encoding Cas proteins Cas5d, Cas6d, Cas7d, Cas3d and Cas10d. Examples of the nucleic acid include RNA such as mRNA and DNA.

In the target sequence-altering method of the present invention, Cas11d may be introduced into the cell or contacted with the isolated nucleic acid comprising the target nucleotide sequence, as an isolated complex comprising Cas11d and the above-mentioned Cas proteins, or Cas11d may be introduced into the cell or contacted with the isolated nucleic acid comprising the target nucleotide sequence, as an isolated single protein. In the target sequence-altering method of the present invention, Cas11d may be also introduced into the cell as a nucleic acid encoding Cas11d. Examples of the nucleic acid include RNA such as mRNA and DNA.

The nucleic acids encoding the Cas proteins and Cas11d may be contained in, for example, a vector. The nucleic acid DNA sequence is preferably operably linked to a regulatory sequence such as a promoter or terminator. When the cell into which the Cas proteins and Cas11d are introduced is a eukaryotic cell, a nuclear localizing signal sequence is preferably added to the nucleic acid sequences encoding the Cas proteins and Cas11d. Two or more or all of nucleic acids encoding the Cas proteins Cas5d, Cas6d, Cas7d and Cas10d, and Cas11d may be contained in a single vector or expression cassette, or may be contained in separate vectors or expression cassettes. The number of the vectors or expression cassettes, and the kinds and combinations of the nucleic acids which are incorporated into each vector or expression cassette are not limited. When two or more nucleic acids encoding the Cas proteins and Cas11d are contained in a single vector or expression cassette, the nucleic acid sequences may be linked to each other, for example via a sequence encoding a self-cleaving peptide, so as to be polycistronically expressed. The two or more nucleic acids encoding the Cas proteins and Cas11d may be linked in any order.

The crRNA may be introduced into the cell as an RNA or as a DNA encoding the crRNA. The crRNA may also be introduced into the cell or contacted with the isolated nucleic acid comprising the target nucleotide sequence, as a complex with the Cas proteins and/or Cas11d. The crRNA or the DNA encoding the crRNA may be contained, for example, in a vector. The RNA or DNA sequence is preferably operably linked to a regulatory sequence such as a promoter or a terminator. The crRNA or the DNA encoding the crRNA may be contained together with the nucleic acids encoding the Cas proteins and/or the nucleic acid encoding Cas11d in the same vector or expression cassette as, or may be contained in a separate vector or expression cassette from the nucleic acids encoding the Cas proteins and/or the nucleic acid encoding Cas11d.

Various kinds of vectors commonly used in the art can be used, and can be appropriately selected depending on the types of cells to which the vectors are introduced or the introduction methods. Examples of the vectors include, but not limited to, plasmid vectors, viral vectors, retroviral vectors, phages, phagemids, cosmids, artificial/minichromosomes, and transposons.

Examples of the regulatory sequences include promoters, enhancers, terminators, internal ribosome entry sites (IRES), polyadenylation signals, poly U sequences, and translation enhancers. The regulatory sequences are not particularly limited, and can be appropriately selected by those skilled in the art considering host cells and the like. For example when the host is a plant cell, examples of the promoter include CaMV35S promoter, 2xCaMV35S promoter, CaMV19S promoter, and NOS promoter. When the host is an animal cell, examples of the promoter include SRα promoter, SV40 promoter, LTR promoter, CMV promoter, RSV promoter, MoMuLV LTR promoter, HSV-TS promoter, human translation elongation factor gene promoter, and CAG chimera synthetic promoter.

The nuclear localizing signal sequence is known in the art, and can be appropriately selected depending on organism species that the cells into which the Cas proteins, Cas11d and crRNA are introduced are derived from. For example, a monopartite nuclear localizing signals or a bipartite nuclear localizing signal may be used.

In the target sequence-altering method of the present invention, in addition to the Cas proteins, Cas11d and crRNA, a donor polynucleotide may be introduced into the cell. The donor polynucleotide comprises at least one donor sequence that comprises alteration desired to be introduced into a target site. The donor polynucleotide may comprise, in addition to the donor sequence, sequences having high homology with the upstream and downstream sequences of the target sequence (preferably, sequences substantially identical to the upstream and downstream sequences of the target sequence) at both ends of the donor sequence. The donor polynucleotide may be a single-stranded or double-stranded DNA. The donor polynucleotide can be appropriately designed by a person skilled in the art based on techniques known in the art.

When the donor polynucleotide is absent in the target sequence-altering method of the present invention, cleavage in the target nucleotide sequence may be repaired by non-homologous end joining (NHEJ) . NHEJ is known to be error-prone, and deletion, insertion, or substitution of one or more nucleotides or a combination thereof may occur during the cleavage repair. Thus, the sequence may be altered at the target sequence site, and thereby frameshift or an immature stop codon is induced to inactivate or knock out the expression of a gene encoded by the target sequence region.

When the donor polynucleotide is present in the target sequence-altering method of the present invention, the donor sequence of the donor polynucleotide is inserted into the target sequence site or replaces the target sequence site by homologous recombination repair (HDR) of the cleaved target nucleotide sequence. As a result, desired alteration is introduced into the target sequence site.

The introduction of the Cas proteins, Cas11d, and crRNA into the cell can be performed by various means known in the art. When the donor polynucleotide is used, the donor polynucleotide may be also introduced into the cell by various means known in the art. Examples of such means include transfection, e.g., calcium phosphate-mediated transfection, electroporation, liposome transfection, etc., virus transduction, lipofection, gene gun, microinjection, Agrobacterium method, Agroinfiltration, and a PEG-calcium method.

The Cas proteins, Cas11d and crRNA, or nucleic acids encoding them, or complexes comprising the Cas protein etc. may be introduced into the cell simultaneously or sequentially. When the donor polynucleotide is used, the donor polynucleotide may be also introduced into the cell simultaneously or sequentially with the Cas proteins, Cas11d and crRNA, or nucleic acids encoding them, or complexes comprising the Cas protein etc.

Upon introduction of the Cas proteins, Cas11d and crRNA, the cell is cultured under suitable conditions for cleavage at the target sequence site. The cell is then cultured under suitable conditions for cell growth and maintenance. The same applies to introduction of the donor polynucleotide. The culture conditions may be suitable for the organism species which the cell into which the Cas proteins, Cas11d and crRNA are introduced is derived from, and can be appropriately determined by a person skilled in the art, for example, based on known cell culture techniques.

According to the target sequence-altering method of the present invention, a site on the target nucleotide sequence is cleaved by the TiD system introduced into the cell, and the target sequence is altered when the cleaved sequence is repaired. For example, the method of altering a target sequence of the present invention can be used for an alteration of a target nucleotide sequence on the genome. A double-stranded DNA on the genome is cleaved and then altered at a target site by the method of altering a target sequence of the present invention. Thus, according to the target sequence-altering method of the present invention, a cell comprising an altered target sequence is produced. Furthermore, when the cell comprising an altered target sequence is a plant cell, a plant comprising an altered target sequence can be produced from the cell. The plant includes a plant body, a tissue, an organ (e.g., root, stem, leaf, etc.), a propagation material (e.g., seed, tuber, etc.), a progeny plant, a cloned plant, and the like. For example, a plant body can be regenerated from a plant cell comprising an altered target sequence to produce a plant body comprising an altered target sequence. The regeneration of a plant body from a plant cell can be performed by a method known in the art. Further, tissues, organs, propagating materials, progeny plants, clones, etc. comprising an altered target sequence can be obtained from the plant body. The target sequence-altering method of the present invention can be also used to produce an animal cell comprising an altered target sequence, and the animal cell can be used to produce an animal comprising an altered target sequence. The animal includes an animal individual, a tissue, an organs, a progeny, a cloned animal, and the like. The animal is preferably a non-human animal. The production of an animal from the animal cell can be performed by a method known in the art. As the animal cell, for example, a germ cell, a fertilized egg, or a pluripotent stem cell is used. An animal individual comprising an altered target sequence may be produced, for example, by introducing the TiD system of the present invention into a fertilized egg, implanting the fertilized egg into the uterus of a non-human animal, and obtaining an offspring. Further, tissues, organs, progenies, clones, etc. comprising an altered target sequence can be obtained from the animal individual.

The target sequence-altering method of the present invention can introduce not only short-range insertions and/or deletions of several bases to several tens of bases but also long-range deletions of several kilobases to several tens of kilobases into a target sequence. Examples of several kilobases to several tens of kilobases include, but not limited to, 1000 to 90000 bases long, preferably 2000 to 80000 bases long, more preferably 2000 to 70000 bases long, and still more preferably 2000 to 60000 bases long, 2000 to 50000 bases long, 2000 to 40000 bases long, 2000 to 30000 bases long, and 2000 to 20000 bases long. Therefore, according to the target sequence-altering method of the present invention, it is possible to delete an entire locus by designing only one guide RNA. Moreover, according to the target sequence-altering method of the present invention, it is possible to completely delete a specific exon even when long introns are present as found in animal genes. Furthermore, according to the target sequence-altering method of the present invention, it is also possible to delete a group of adjacent genes collectively.

According to the target sequence-altering method of the present invention, base deletions can be introduced upstream or downstream of the PAM sequence, or both upstream and downstream of the PAM sequence (i.e., bi-directional deletions).

### (8) Target gene expression-regulating method of the present invention

Further, when at least a partial sequence of a target gene or a transcription regulatory sequence (e.g., a transcription factor binding sequence, a promoter sequence, an enhancer sequence, etc.) for a target gene is selected as the target nucleotide sequence in the target sequence-targeting method or the target sequence-altering method of the present invention, the transcription of the target gene can be suppressed, thereby suppressing the expression of the target gene. Further, when at least a partial sequence of a transcription regulatory sequence for a target gene is selected as the target nucleotide sequence in the target sequence-targeting method of the present invention, and a fusion protein of the Cas proteins and a gene expression regulation module (for example, a transcription activation module, or a transcription repression module of a transcription factor) is used, the transcription of the target gene can be regulated (activated or inactivated), thereby regulating (amplifying or repressing) the expression of the target gene. Thus, the present invention provides a method for regulating the expression of a target gene.

In the target gene expression-regulating method of the present invention, at least a partial sequence of a target gene or a transcription regulatory sequence for a target gene is selected as the target nucleotide sequence, and a crRNA comprising a sequence capable of forming a base pair with the selected sequence is used. The target gene expression-regulating method of the present invention comprises suppressing the transcription of the target gene by binding of a complex of the Cas proteins and the crRNA to the target nucleotide sequence when the nucleotide sequence is targeted by the target sequence-targeting method of the present invention. In such a case, though the target gene sequence is not cleaved, the function of the target gene region or the transcription or expression of the target gene is inhibited by binding of the complex of the Cas proteins and the crRNA to the target nucleotide sequence. In another aspect, the target gene expression-regulating method of the present invention comprises suppressing the transcription of the target gene by targeting and cleaving the nucleotide sequence by the target sequence-altering method of the present invention. The target gene expression-regulating method of the present invention may be performed in vitro, in vivo or ex vivo.

The Cas proteins, Cas11d and crRNA, the method of introducing them into cells, and the cell culture during and after introduction, etc. are as described in "(6) Target sequence-targeting method of the present invention" and "(7) Target sequence-altering method of the present invention".

### (9) Complex of the present invention

The complex of the present invention comprises the above-mentioned Cas proteins, Cas11d, and crRNA. The present invention particularly provides a complex comprising Cas5d, Cas6d, Cas7d, Cas10d, Cas11d, and crRNA, and a complex comprising Cas5d, Cas6d, Cas7d, Cas3d, Cas10d, Cas11d, and crRNA. Further, the present invention provides a complex comprising a fusion protein comprising Cas5d, Cas6d, Cas7d, Cas10d and a functional polypeptide, Cas11d and crRNA. Further provided is a DNA molecule encoding the complex as described above. The complex of the present invention can be used in the target sequence-targeting method, the target sequence-altering method, and the target gene expression-regulating method of the present invention. For example, a target sequence on the genome of a cell can be altered by introducing a complex comprising Cas5d, Cas6d, Cas7d, Cas3d and Cas10d and a complex comprising Cas11d and the crRNA into the cell to allow the complexes to function within the cell. A target sequence in a cell can be targeted and the expression of a target gene can be regulated by introducing a complex comprising Cas5d, Cas6d, Cas7d and Cas10d and a complex comprising Cas11d and the crRNA into the cell to allow the complexes to function within the cell.

The complex of the present invention can be produced in vitro, in vivo or ex vivo by a conventional method. For example, nucleic acids encoding the Cas proteins, a nucleic acid encoding Cas11d, and the crRNA or a DNA encoding the crRNA may be introduced into a cell to allow the complex to form in the cell.

Examples of the complex of the present invention include, but not limited to, a complex comprising Cas5d (SEQ ID NO: 2), Cas6d (SEQ ID NO: 3), Cas7d (SEQ ID NO: 4) and Cas10d (SEQ ID NO: 5), and Cas11d (SEQ ID NO: 6) that are derived from *Microcystis aeruginosa,* and a crRNA consisting of a sequence shown by GUUCCAAUUAAUCUUAAGCCCUAUUAGGGAUUGAAACNNNNNNNNNNNNNNNNNNNNNN NNNNNNNNNNNNNGUUCCAAUUAAUCUUAAGCCCUAUUAGGGAUUGAAAC (SEQ ID NO:7; N is any nucleotide constituting a sequence complementary to a target nucleotide sequence), and a complex comprising Cas 5d (SEQ ID NO: 2), Cas6d (SEQ ID NO: 3), Cas7d (SEQ ID NO: 4), Cas3d (SEQ ID NO: 1) and Cas10d (SEQ ID NO: 5), and Cas11d (SEQ ID NO: 6) that are derived from *Microcystis aeruginosa,* and a crRNA consisting of a sequence shown by GUUCCAAUUAAUCUUAAGCCCUAUUAGGGAUUGAAACNNNNNNNNNNNNNNNNNNNNNN NNNNNNNNNNNNNGUUCCAAUUAAUCUUAAGCCCUAUUAGGGAUUGAAAC (SEQ ID NO:7; N is any nucleotide constituting a sequence complementary to a target nucleotide sequence). In the crRNA sequence, the number of N may be varied within a range of 10 to 70, preferably 20 to 50, more preferably 25 to 45, still more preferably 30 to 40, and still more preferably 32 to 37.

### (10) Expression vector of the present invention

The present invention further provides an expression vector containing nucleic acids encoding the Cas proteins Cas5d, Cas6d, Cas7d and Cas10d, a nucleic acid encoding Cas11d, and the crRNA or a DNA encoding the crRNA, and an expression vector containing nucleic acids encoding the Cas proteins Cas3d, Cas5d, Cas6d, Cas7d and Cas10d, a nucleic acid encoding Cas11d, and the crRNA or a DNA encoding the crRNA.

The vector of the present invention is a vector for introducing the Cas proteins, Cas11d and the crRNA into the cell, as described in "(6) Target sequence-targeting method of the present invention", "(7) Target sequence-altering method of the present invention", and "(8) Target gene expression-regulating method of the present invention". After the introduction of the vector into the cell, the Cas proteins, Cas11d and the crRNA are expressed in the cell. The vector of the present invention may be also a vector in which the target sequence contained in the crRNA is replaced by any sequence containing a restriction site. Such a vector is used after incorporating a desired target nucleotide sequence into the restriction site. Any sequence may be, for example, a spacer sequence present on the CRISPR type I-D locus or a part of the spacer sequence.

The nucleic acids encoding the Cas proteins, the nucleic acid encoding Cas11d, and the crRNA or the DNA encoding the crRNA may be contained in the same vector, or may be contained separately in two or more vectors.

### (11) Kit of the present invention

The kit of the present invention is a kit for use in the target sequence-targeting method, the target sequence-altering method and the target gene expression-regulating method of the present invention. The kit of the present invention comprises the Cas proteins Cas5d, Cas6d, Cas7d and Cas10d, or nucleic acids encoding these proteins, Cas11d or a nucleic acid encoding Cas11d, and the crRNA or a DNA encoding the crRNA, or the Cas proteins Cas3d, Cas5d, Cas6d, Cas7d and Cas10d, or nucleic acids encoding these proteins, Cas11d or a nucleic acid encoding Cas11d, and the crRNA or a DNA encoding the crRNA. The nucleic acids encoding the Cas proteins and Cas11d and/or the DNA encoding the crRNA may be contained in a vector system or an expression cassette system. The components of the kit of the present invention are as described in above sections (2) to (7).

The present invention further provides a method for improving the efficiency of targeting and altering a target nucleotide sequence utilizing the TiD system, characterized by using Cas11d, and a composition for improving the efficiency of targeting and altering a target nucleotide sequence utilizing the TiD system, comprising Cas11d. In the above-described method, Cas11d is introduced into a cell comprising the target sequence. The implementation, components, etc. of the above-described method and composition are as described in above sections (1) to (7).

Hereinafter, examples of the present invention are shown. However, the present invention is not limited to the examples.

### Examples

As one embodiment, a group of genes (Cas3d, Cas5d, Cas6d, Cas7d, Cas10d) derived from the TiD locus derived from *Microcystis aeruginosa* were cloned and then used. Based on amino acid sequence information (SEQ ID NOs: 1 to 5) of Cas3d, Cas5d, Cas6d, Cas7d, Cas10d from *Microcystis aeruginosa,* a DNA sequence encoding each Cas protein was artificially synthesized. For processing and construction of DNA sequences in Examples, artificial gene chemical synthesis, PCR, restriction enzyme treatment, ligation, or a Gibson Assembly method was used. In addition, the Sanger method or a next generation sequencing method was used to determine nucleotide sequences.

For a Cas10d C-ter sequence, a region of 483 bases 5'-upstream of the stop codon of Cas10d was cloned and then used.

A target sequence used is as described below. The PAM is GTC.
AAVS1_GTC_70-107(+): cctagtggccccactgtggggtggaggggacagat (SEQ ID NO: 20)

### <Method>

### (1) Vector construction

PCR amplification for cloning of gene fragments was performed using PrimeSTAR Max (TaKaRa). Cloning for assembly was performed using Quick ligation kit (NEB), NEBuilder HiFi DNA Assembly (NEB), and Multisite gateway Pro (Thermo Fisher Scientific).

### (1-1) Mammalian vector

The Cas effector genes (Cas3d, Cas5d, Cas6d, Cas7d, and Cas10d) and Cas11d that were optimized for human codons were synthesized together with a SV40 nuclear localizing signal (NLS) (SEQ ID NO: 21: KKKKRK) at their N-termini [gBlocks (registered trademark)] (IDT), assembled, and separately cloned into pEFs vectors (Lopez-Perrote et al, 2016, Nucleic Acids Res, 44:1909-1923. doi:10.1093/nar/gkv1527) to obtain single Cas expression vectors: pEFs-myc-SV40NLS-Cas3d, pEFs-myc-SV40NLS-Cas5d, pEFs-myc-SV40NLS-Cas6d, pEFs-myc-SV40NLS-Cas7d, pEFs-myc-SV40NLS-Cas10d, and pEFs-myc-SV40NLS-Cas11d. A myc tag was fused to each Cas protein.

For a crRNA expression vector, a DNA fragment containing a repeat-spacer-repeat sequence (SEQ ID NO: 22) was artificially synthesized, and cloned into pEX-A2J1 (Eurofins Genomics) under the control of a human U6 promoter to obtain pAEX-hU6crRNA. For insertion of a gRNA sequence, two oligonucleotides containing the target sequence were annealed, and cloned into the crRNA expression vector using Golden Gate cloning with restriction enzyme BsaI (NEB).

**[Table 1]**

| | |
|---|---|
| SEEQ ID NO: 22 Pre-mature type | GTTCCAATTAATCTTAAGCCCTATTAGGGATTGAAACgga gaccctcaattgtcggtctcGTTCCAATTAATCTTAAGCC CTATTAGGGATTGAAACTTTTTTTT |
| | Uppercase letters indicate repeat sequences. Lowercase letters indicate a cloning site. The BsaI site is underlined. A polyT sequence for transcription termination is indicated by "TTTTTTTT". |

### (1-2) Luciferase reporter assay plasmid

For luciferase (luc) reporter assay, NanoLUxxUC expression vectors were constructed. First, NLUxxUC_Block1 and NLUxxUC_Block2 DNA fragments were synthesized (IDT). NLUxxUC_Block1 contains 351 bp from the 5' end of NanoLUC^{™} (registered trademark) gene (Promega) sequence and a multiple cloning site, and an XbaI site was attached to the 5' end. NLUxxUC_Block2 contains 465 bp from the 3' end of the NanoLUC gene, and an XhoI site was attached to the 3' end. These fragments were assembled and cloned into pCAG-EGxxFP vectors (Addgene, #50716). NLUxxUC_Block1 and NLUxxUC_Block2 were removed from pCAG-NLUxxUC vectors by XbaI and BamHI digestion and by XbaI and EcoRI digestion respectively to construct each split-type NLUxxUC reporter. Each digested vector was assembled with a multiple cloning site to obtain pCAG-NLUxxUC_Block1 and pCAG-NLUxxUC_Block2.

### (2) Cell culture and transfection

Human embryonic kidney cell line 293T (HEK293T, RIKEN BRC) was cultured in a Dulbecco's modified Eagle's medium (DMEM) supplemented with 10% fetal bovine serum (Thermo Fisher Scientific), GlutalMAX (registered trademark) supplement (Thermo Fisher Scientific), 100 units/mL penicillin, and 100 ug/mL streptomycin at 37°C for 60 minutes with 5% CO₂ incubation. HEK293T cells were seeded onto a 6-well plate (Corning, USA) the day before transfection, and transfected using TurboFect Transfection Reagent (Thermo Fisher Scientific) following the manufacturer's protocol. A total of 4 µg of plasmids extracted using NucleoSpin (registered trademark) Plasmid Transfection-grade kit (Macherey-Nagel, Germany) were used in each well of the 6-well plate. Forty-eight hours after, transfected cells were collected for mutation analysis.

### (3) Luciferase reporter assay

HEK293T cells were seeded onto a 96-well plate (Corning) at a density of 2.0 × 10⁴ cells/well the day before transfection, and transfected using TurboFect Transfection Reagent (Thermo Fisher Scientific) following the manufacturer's protocol. A total of 200 ng of plasmid DNAs including (1) a pGL4.53 vector encoding Flue gene (Promega, USA), (2) a pCAG-nLUxxUC vector interrupted by insertion of the target DNA fragment, and (3) plasmid DNAs encoding TiD components were used in each well of the 96-well plate. NanoLuc and Flue luciferase activities were measured 3 days after transfection using Nano-Glo (registered trademark) Dual-Luciferase (registered trademark) Reporter Assay System (Promega). The firefly (Flue) activity was used as an internal control. A NanoLuc/Fluc ratio was calculated for each sample, and compared with the NanoLuc/Fluc ratio of a control sample that was transfected with a non-targeting gRNA. Relative NanoLuc/Fluc activity was used to evaluate gRNA activity. Experiments were repeated three times independently, and similar results were obtained.

### (4) DNA deletion analysis by long-range PCR

For detection of DNA deletions in HEK293T cells, long-range PCR was performed, and a pool of long-range PCR products was cloned. First, a genomic DNA was extracted from HEK293T cells using Geno Plus (registered trademark) Genomic DNA Extraction Miniprep System (Viogene-BioTek, Taiwan). Next, nested PCR was performed to specifically amplify long-range DNA regions. Specifically, target DNA regions were amplified by using the extracted genomic DNA as a template and using specific primer sets for long-range PCR that were designed to amplify target DNA regions of various lengths (10 kb to 19 kb). The first PCR reaction was performed using KOD ONE Master Mix (TOYOBO, Osaka, Japan) under the following conditions: 35 cycles of 10 sec at 98°C, 5 sec at 60°C, and 50 sec (amplicon: 15-20 kb) or 150 sec (amplicon: 10-15 kb) or 200 sec (amplicon: <10 kb) at 68°C. PCR products were diluted 100-10,000 times and then used as templates for the nested PCR. The nested PCR was also performed under the same conditions as described above. PCR products were separated by electrophoresis on a 1% agarose gel and visualized by staining with GelRed (registered trademark) Nucleic Acid Gel Stain (Biotium). The nested PCR products were pooled and purified using Monofas (registered trademark) DNA purification Kit I (GL Sciences, Japan). A mixture of the purified PCR products was cloned into a pMD20-T vector using Mighty TA-cloning Kit (Takara Bio, Japan). Clones were picked up and analyzed by Sanger sequencing using M13 Uni and M13 RV primers. Results of Sanger sequencing were analyzed using BLATN searches and ClustalW program to identify DNA deletions.

### Example 1: Identification of Cas11e-like sequence (Cas11d) in Cas10d sequence

Of the Cas effector proteins from *M. aeruginosa,* the sequence of Cas10d was compared with CRISPR type I-E Cas11e sequences (from *Escherichia coli, Acetobacter pasteurianus, Acidimicrobium ferrooxidans, Amycolatopsis mediterranei, Bifidobacterium animalis, Cellulomonas fimi, Coriobacterium glomerans, Cyanothece (Gloeothece citriformis)* PCC7424, *Desulfococcus oleovorans, Erwinia amylovora, Frankia alni, Geobacter sulfurreducens, Kitasatospora setae,* and *Lactobacillus fermentum)* by alignment analysis using CLASTAL W program. As a result, it was found that alpha-helix regions characteristic of Cas11e are conserved at several positions in the C-terminal region of Cas10d (see FIG. 1). In addition, various type I-D cas10d C-ter sequences (from *Anabaena cylindrica, Calothrix* PCC6303 (*Calothrix parietina*)*, Crinalium epipsammum, Cyanothece* PCC7424 (*Gloeothece citriformis*), *Gloeobacter kilaueensis, Gloeocapsa sp.* PCC7428, *Halothece* PCC7418, *Methanospirillum hungatei, Nostoc sp.* NIES-2111, *Rivularia sp.* PCC7116, *Stanieria cyanosphaera, Synechocystis sp.* PCC6803) were compared with Cas11e sequences by alignment analysis.

### Example 2: Analysis of effect of Cas10d C-ter (Cas11d) expression on genome editing activity in cell

To analyze the effect of Cas11d expression on the genome editing activity of TiD in eukaryotes, a gene sequence region (up to 483 bases upstream from the stop codon of Cas10d gene) in the Cas10d gene from *Microcystis aeruginosa* PC9808 which was believed to correspond to a Cas11d sequence was cloned to construct a vector for animal cell expression, which was used as a vector for Cas11d expression. The Cas11d expression vector was introduced into HEK293 cells simultaneously with the Cas3d, Cas5d, Cas6d, Cas7d, Cas10d and gRNA expression vectors, and the genome editing activity was analyzed by Luc reporter assay. A NanoLuc luciferase containing 300 bp homology arms separated by a stop codon and a human AAVS1 gene fragment containing the TiD target site was used as a recombination reporter. HEK293T cells were transfected simultaneously with each of the single Cas expression vectors, the TiD crRNA expression vector, and the LUC reporter vector into which the target sequence was introduced, and then endonuclease cleavage was detected by luminescence 72 hours after transfection.

As a result, when Cas11d was further expressed, the genome editing activity was increased by 2.5 times as compared with the genome editing activity by the expression vectors of Cas3d, Cas5d, Cas6d, Cas7d, Cas10d and crRNA (FIG. 2). Even when the length of crRNA used for targeting was varied, i.e., a crRNA for targeting a shortened target sequence of 30 bases in length (SEQ ID NO: 23: 5'-CCTAGTGGCCCCACTGTGGGGTGGAGGGGA-3') was used, the effect of Cas11d expression was observed (FIG. 2).

### Example 3: Analysis of human genome editing by expression of Cas10d C-ter (Cas11d)

In this Example, it was analyzed what kind of mutations were induced in the target on the human genome by the Cas11d expression. HEK293T cells were transfected simultaneously with the Cas11d expression vector, the gRNA expression vector incorporating the human AAVS gene-targeting gRNA AAVS GTC_70-107 (35b) that was used for the Luc reporter assay, and each of the single Cas expression vectors. A DNA fragment was amplified from a total DNA of the HEK293T cells transfected with the TiD vectors by PCR using a primer set for amplifying 10-19 kb including the vicinity of the AAVS target site, and the resulting PCR products were cloned and sequenced by the Sanger method.

As the primers, F1 (SEQ ID NO: 24: 5'-CTTAGCATAATGTCCTCAAGATACATCTAC-3') and R1 (SEQ ID NO: 25: 5'-GATATGTAACCATTATTCTAGATGGCTATG-3'), and primers F2 (SEQ ID NO: 26: 5'-GGGTCCAAGGGAAAAGGAGGACTGATCC-3') and R2 (SEQ ID NO: 27: 5'-ATAAACACAAACTCATAAACAACATACATC-3') were used.

First, PCR was performed using primers F1 and R1 as shown in FIG. 3A to obtain an amplified DNA, which was referred to as a 1st-PCR product. Next, the 1st-PCR product was diluted 20 to 50 times, and subjected to PCR using primers F2 and R2 as shown in FIG. 3A and then electrophoretic analysis. Results are shown in FIG. 3B. Lane 1 indicates PCR products using a DNA derived from the wild-type HEK293 cell that does not express TiD. Lanes 2 and 3 indicate results of experiments in which the TiD that did not comprise the Cas11d expression vector was introduced. Lane 2 indicates a result of PCR using a gRNA comprising a sequence corresponding to a non-specific sequence (SEQ ID NO: 28: 5'-AAATAAATAGCGGTCGGGTGCCCCGAATTTCACAT-3') in place of the target sequence. Lane 3 indicates a result of PCR using a gRNA comprising a sequence corresponding to the target sequence AAVS GTC_70-107 (35b) . Lanes 4 and 5 indicate results of experiments in which the TiD comprising the Cas11d expression vector was introduced. Lane 4 indicates a result of PCR using a gRNA comprising a sequence corresponding to the non-specific sequence in place of the target sequence. Lane 5 indicates a result of PCR using a gRNA comprising a sequence corresponding to AAVS GTC_70-107 (35b).

As a result, introduction of TiD resulted in long-range deletions over 6 kb at the target site. Interestingly, the results of sequence analysis showed that longer range deletions occurred in the experiments comprising introduction of the Cas11d expression vector than in the experiments without introduction of the Cas11d expression vector (FIG. 3C and 3D).

### Sequence Listing Free text

SEQ ID NO:1; Microcystis aeruginosa Cas3d amino acid sequence
SEQ ID NO:2; Microcystis aeruginosa Cas5d amino acid sequence
SEQ ID NO:3; Microcystis aeruginosa Cas6d amino acid sequence
SEQ ID NO:4; Microcystis aeruginosa Cas7d amino acid sequence
SEQ ID NO:5; Microcystis aeruginosa Cas10d amino acid sequence
SEQ ID NO:6; Microcystis aeruginosa Cas11d amino acid sequence
SEQ ID NO:7; TiDcrRNA containing repeat (37b) and spacer (35b of N). N is any nucleotide constituting a sequence that forms base pairs with a target nucleotide sequence
SEQ ID NO:8; Anabaena cylindrica Cas11d amino acid sequence
SEQ ID NO:9; Calothrix PCC6303 (Calothrix parietina) Cas11d amino acid sequence
SEQ ID NO:10; Crinalium epipsammum Cas11d amino acid sequence
SEQ ID NO:11; Cyanothece PCC7424 (Gloeothece citriformis) Cas11d amino acid sequence
SEQ ID NO:12; Gloeobacter kilaueensis Cas11d amino acid sequence
SEQ ID NO:13; Gloeocapsa sp. PCC7428 Cas11d amino acid sequence
SEQ ID NO:14; Halothece PCC7418 Cas11d amino acid sequence
SEQ ID NO:15; Methanospirillum hungatei Cas11d amino acid sequence
SEQ ID NO:16; Nostoc sp. NIES-2111 Cas11d amino acid sequence
SEQ ID NO:17; Rivularia sp. PCC7116 Cas11d amino acid sequence
SEQ ID NO:18; Stanieria cyanosphaera Cas11d amino acid sequence
SEQ ID NO:19; Synechocystis sp. PCC6803 Cas11d amino acid sequence
SEQ ID NO:20; Target sequence (35b)
SEQ ID NO:21; Monopartite nuclear localizing signal (NLS) amino acid sequence
SEQ ID NO:22; DNA fragment for pre-mature crRNA
SEQ ID NO:23; Target sequence (30b)
SEQ ID NO:24; Primer F1
SEQ ID NO:25; Primer R1
SEQ ID NO:26; Primer F2
SEQ ID NO:27; Primer R2
SEQ ID NO:28; Non-specific sequence
SEQ ID NO:29; Escherichia coli Cas11e amino acid sequence
SEQ ID NO:30; Acetobacter pasteurianus Cas11e amino acid sequence
SEQ ID NO:31; Acidimicrobium ferrooxidans Cas11e amino acid sequence
SEQ ID NO:32; Amycolatopsis mediterranei Cas11e amino acid sequence
SEQ ID NO:33; Bifidobacterium animalis Cas11e amino acid sequence
SEQ ID NO:34; Cellulomonas fimi Cas11e amino acid sequence
SEQ ID NO:35; Coriobacterium glomerans Cas11e amino acid sequence
SEQ ID NO:36; Cyanothece (Gloeothece citriformis) PCC7424 Cas11e amino acid sequence
SEQ ID NO:37; Desulfococcus oleovorans Cas11e amino acid sequence
SEQ ID NO:38; Erwinia amylovora Cas11e amino acid sequence SEQ ID NO:39; Frankia alni Cas11e amino acid sequence
SEQ ID NO:40; Geobacter sulfurreducens Cas11e amino acid sequence
SEQ ID NO:41; Kitasatospora setae Cas11e amino acid sequence
SEQ ID NO:42; Lactobacillus fermentum Cas11e amino acid sequence

## Claims

1. A method for targeting a target nucleotide sequence, the method comprising introducing into a cell:
(i) CRISPR type I-D Cas proteins Cas5d, Cas6d and Cas7d, and a polypeptide containing the N-terminal HD domain of Cas10d, or nucleic acids encoding the proteins and the polypeptide,
(ii) a polypeptide that does not contain the N-terminal HD domain of Cas10d and contains a C-terminal partial sequence of Cas10d, or a nucleic acid encoding the polypeptide, and
(iii) a crRNA containing a sequence capable of forming a base pair with the target nucleotide sequence, or a DNA encoding the crRNA.

2. A method for altering a target nucleotide sequence, the method comprising introducing into a cell:
(i) CRISPR type I-D Cas proteins Cas3d, Cas5d, Cas6d and Cas7d, and a polypeptide containing the N-terminal HD domain of Cas10d, or nucleic acids encoding the proteins and the polypeptide,
(ii) a polypeptide that does not contain the N-terminal HD domain of Cas10d and contains a C-terminal partial sequence of Cas10d, or a nucleic acid encoding the polypeptide, and
(iii) a crRNA containing a sequence capable of forming a base pair with the target nucleotide sequence, or a DNA encoding the crRNA.

3. The method according to claim 1 or 2, which is for regulating the transcription of a target gene, and wherein the target nucleotide sequence is at least a partial sequence of the target gene.

4. The method according to any one of claims 1 to 3, wherein the C-terminal partial sequence of Cas10d is a sequence consisting of 100 to 400 amino acids.

5. The method according to any one of claims 1 to 4, wherein the cell is a eukaryotic cell.

6. The method according to any one of claims 2 to 5, wherein the alteration is nucleotide deletion, insertion or substitution.

7. A complex comprising:
(i) CRISPR type I-D Cas proteins Cas5d, Cas6d and Cas7d, and a polypeptide containing the N-terminal HD domain of Cas10d,
(ii) a polypeptide that does not contain the N-terminal HD domain of Cas10d and contains a C-terminal partial sequence of Cas10d, and
(iii) a crRNA containing a sequence capable of forming a base pair with the target nucleotide sequence.

8. The complex according to claim 7, further comprising Cas3d.

9. The complex according to claim 7 or 8, wherein the C-terminal partial sequence of Cas10d is a sequence consisting of 100 to 400 amino acids.

10. A vector containing:
(i) nucleic acids encoding CRISPR type I-D Cas proteins Cas5d, Cas6d and Cas7d, and a polypeptide containing the N-terminal HD domain of Cas10d,
(ii) a nucleic acid encoding a polypeptide that does not contain the N-terminal HD domain of Cas10d and contains a C-terminal partial sequence of Cas10d, and
(iii) a crRNA comprising a sequence capable of forming a base pair with the target nucleotide sequence, or a DNA encoding the crRNA.

11. The vector according to claim 10, further containing a nucleic acid encoding Cas3d.

12. The expression vector according to claim 10 or 11, wherein the nucleic acids of (i) to (iii), or the nucleic acids of (i) to (iii) and the nucleic acid encoding Cas3d are contained in a single vector or two or more vectors.

13. The vector according to claim 11 or 12, wherein the C-terminal partial sequence of Cas10d is a sequence consisting of 100 to 400 amino acids.

14. A DNA molecule encoding the complex according to any one of claims 7 to 9.

15. A kit for targeting a target nucleotide sequence, comprising:
(i) CRISPR type I-D Cas proteins Cas5d, Cas6d and Cas7d, and a polypeptide containing the N-terminal HD domain of Cas10d, or nucleic acids encoding the proteins and the polypeptide,
(ii) a polypeptide that does not contain the N-terminal HD domain of Cas10d and contains a C-terminal partial sequence of Cas10d, or a nucleic acid encoding the polypeptide, and
(iii) a crRNA containing a sequence capable of forming a base pair with the target nucleotide sequence, or a DNA encoding the crRNA.

16. A kit for altering a target nucleotide sequence, comprising:
(i) CRISPR type I-D Cas proteins Cas3d, Cas5d, Cas6d and Cas7d, and a polypeptide containing the N-terminal HD domain of Cas10d, or nucleic acids encoding the proteins and the polypeptide,
(ii) a polypeptide that does not contain the N-terminal HD domain of Cas10d and contains a C-terminal partial sequence of Cas10d, or a nucleic acid encoding the polypeptide, and
(iii) a crRNA containing a sequence capable of forming a base pair with the target nucleotide sequence, or a DNA encoding the crRNA.

17. The kit according to claim 15 or 16, wherein the C-terminal partial sequence of Cas10d is a sequence consisting of 100 to 400 amino acids.

18. A method for improving targeting efficiency in targeting a target nucleotide sequence using a CRISPR type I-D system, the method comprising using a polypeptide that does not contain the N-terminal HD domain of Cas10d and contains a C-terminal partial sequence of Cas10d, or a nucleic acid encoding the polypeptide.

19. A method for improving alternation efficiency in altering a target nucleotide sequence using a CRISPR type I-D system, the method comprising using a polypeptide that does not contain the N-terminal HD domain of Cas10d and contains a C-terminal partial sequence of Cas10d, or a nucleic acid encoding the polypeptide.

20. A composition for improving targeting efficiency in targeting a target nucleotide sequence using a CRISPR type I-D system, comprising a polypeptide that does not contain the N-terminal HD domain of Cas10d and contains a C-terminal partial sequence of Cas10d, or a nucleic acid encoding the polypeptide.

21. A composition for improving alteration efficiency in altering a target nucleotide sequence using a CRISPR type I-D system, comprising a polypeptide that does not contain the N-terminal HD domain of Cas10d and contains a C-terminal partial sequence of Cas10d, or a nucleic acid encoding the polypeptide.

22. A method for producing a cell comprising an altered target nucleotide sequence, the method comprising introducing into a cell:
(i) CRISPR type I-D Cas proteins Cas3d, Cas5d, Cas6d and Cas7d, and a polypeptide containing the N-terminal HD domain of Cas10d, or nucleic acids encoding the proteins and the polypeptide,
(ii) a polypeptide that does not contain the N-terminal HD domain of Cas10d and contains a C-terminal partial sequence of Cas10d, or a nucleic acid encoding the polypeptide, and
(iii) a crRNA containing a sequence capable of forming a base pair with the target nucleotide sequence, or a DNA encoding the crRNA.

23. A method for producing a plant comprising an altered target nucleotide sequence, the method comprising producing a plant cell comprising the altered target nucleotide sequence by the method according to claim 22.

24. A method for producing a non-human animal comprising an altered target nucleotide sequence, the method comprising producing a non-human animal cell comprising the altered target nucleotide sequence by the method according to claim 22.

25. A method for targeting a target nucleotide sequence, the method comprising bringing:
(i) CRISPR type I-D Cas proteins Cas5d, Cas6d and Cas7d, and a polypeptide containing the N-terminal HD domain of Cas10d,
(ii) a polypeptide that does not contain the N-terminal HD domain of Cas10d and contains a C-terminal partial sequence of Cas10d, and
(iii) a crRNA containing a sequence capable of forming a base pair with the target nucleotide sequence, or a DNA encoding the crRNA,
into contact with an isolated nucleic acid comprising the target nucleotide sequence.

26. A method for altering a target nucleotide sequence, the method comprising bringing:
(i) CRISPR type I-D Cas proteins Cas3d, Cas5d, Cas6d and Cas7d, and a polypeptide containing the N-terminal HD domain of Cas10d,
(ii) a polypeptide that does not contain the N-terminal HD domain of Cas10d and contains a C-terminal partial sequence of Cas10d, and
(iii) a crRNA containing a sequence capable of forming a base pair with the target nucleotide sequence, or a DNA encoding the crRNA,
into contact with an isolated nucleic acid comprising the target nucleotide sequence.
